# EUROPEAN PATENT SPECIFICATION

(11) **EP 3 899 540 B1**
(45) Date of publication and mention of the grant of the patent: **09.12.2026**
(21) Application number: 19831684.6
(22) Date of filing: 18.12.2019
(51) Int. Cl.: G01N 33/543, G01N 33/569

(54) **SYSTEM FOR CAPTURING CELLS**
SYSTEM ZUR ERFASSUNG VON ZELLEN
SYSTÈME POUR CAPTURER DES CELLULES

(30) Priority: 18.12.2018 GB 201820617
(43) Date of publication of application: 27.10.2021
(73) Proprietor: Greindx AS, 0349 Oslo (NO)
(72) Inventor: LICHTENSTEIN, Amir, 0349 Oslo (NO); AXELROD, Noel, 0349 Oslo (NO); SOLESVIK, Ove Vincent, 0349 Oslo (NO); EZERZER, Chai, 6960000 Tel Aviv (IL)
(74) Representative: Dehns
(86) International application number: PCT/EP2019/086104
(87) International publication number: WO 2020/127591

(56) References cited:
- WO-A1-2012/007537
- WO-A1-2015/191684
- WO-A1-2017/074934
- US-A- 6 063 589
- US-A1- 2002 119 578
- US-B2- 9 856 535

## Description

The present invention relates to a cell extraction device and to a cell analysis system and methods for cell extraction using the cell analysis system.

Cellular heterogeneity within diseased tissue is a key indication for the progression of diseases and can therefore affect the diagnosis, prognosis and treatment of a patient. During diagnostics it is often found that the ensemble of cells obscures the response of aberrant cells, leading to false negative results, and consequently the spread of a disease and/or the response to therapy is often missed. For example, dynamic changes in the phenotype and genotype of circulating tumor cells (CTC) in Androgen Receptor (AR) population should be fed back into the clinical application to benefit cancer treatment of "Castration Resistant Prostate Cancer" (CRPC).

Single-cell assays help to reveal the diversity of cellular behavior which is often obscured in common data averaging of measurements obtained from a cell population as a whole. Studies that resolve the cell heterogeneity reveal whether there is a range of responses towards a treatment and unravel the response of an aberrant rare cell that is sometimes lost amongst an array of measurements. By unraveling cell heterogeneity, it may help in the detection and treatment of the spread of cancer, help to understand resistance of different cancers to therapy, help the heterogeneity in the uptake of drug delivery vehicles etc.

To identify and understand aberrant behavior, single-cell technologies require advancement beyond their current ability to phenotypically identify rare cells, and to reveal the transition processes and functional diversity of these cells. Examples of functional diversity that reflect heterogeneity in rare cells include ALK rearrangement status on CTC of non-small-cell-lung-cancer (NSCLC), adult stem cells that are believed to be responsible for observed variations in the efficiency of tissue repair, and maternal/fetal cells that have been postulated to play a role in the variations in immune response that mothers exhibit before and after childbirth. The rarity of CTC in the peripheral blood (a few to hundreds per mL of whole blood) and the abundance of other cells in the blood (erythrocytes ~10⁹ per mL of whole blood and leukocytes ~10⁶ per mL of whole blood) makes their enumeration an important indicator for cancer and cancer treatment alike. Current technologies only offer slow, cumbersome and expensive processes and financial limitations rule out regular monitoring of large populations worldwide.

WO 2017/074934 A1 discloses single cell capture with chips.

It is therefore desirable to provide an improved system for capturing cells, particularly rare cells.

The invention concerns a cell extraction device comprising:
a plurality of cell extraction modules (34, 44, 54) arranged in a single layer array, the single layer array having a cell-receiving side wherein an opening of at least one of the plurality of cell extraction modules on the cell-receiving surface is configured to receive and retain a single target cell from a fluid sample, and a fluid-evacuating side wherein an opening of the or each of the plurality of cell extraction modules on the fluid-evacuating side is configured to allow fluid from the fluid sample to be evacuated from the cell extraction device,
wherein at least one of the plurality of cell extraction modules comprises a micropore capillary (38, 48, 58) configured at a first end that is open on the cell-receiving side to receive a target cell, and configured at a second end that is open on the fluid-evacuating side to allow fluid to pass through, and
wherein at least one micropore capillary is configured with the first end wider than the second end, wherein the dimension of the first end of the capillary corresponds to or is smaller than the dimension of a single target cell, and the second end is so dimensioned to be smaller than the dimension of a single target cell to prevent a target cell received by the first end to pass through.

The invention also concerns a cell analysis system comprising:
a cell extraction device according to the invention as described above; and
a rotating device coupled to the cell extraction device configured to rotate the cell extraction device at a predetermined angular speed such that a centrifugal force is exerted on the fluid sample placed on the cell extraction device.

The invention also concerns a method of cell extraction using a cell analysis system of the invention as described above comprising:
applying a fluid sample onto the cell extraction device;
activating the rotation device to rotate the cell extraction device at a predetermined angular speed such that a centrifugal force is exerted on the fluid sample applied to the cell extraction device.

In one aspect, there is provided a cell extraction device comprising a plurality of cell extraction modules arranged in a single layer array, the single layer array having a cell-receiving side wherein an opening of at least one of the plurality of cell extraction modules on the cell-receiving surface is configured to receive and retain a single target cell from a fluid sample, and a fluid-evacuating side wherein an opening of the or each of the plurality of cell extraction modules on the fluid-evacuating side is configured to allow fluid from the fluid sample to be evacuated from the cell extraction device, wherein at least one of the plurality of cell extraction modules comprises a micropore capillary configured at a first end that is open on the cell-receiving side to receive a target cell, and configured at a second end that is open on the fluid-evacuating side to allow fluid, but not target cells, to pass through, and wherein at least one micropore capillary is configured with the first end wider than the second end, wherein the dimension of the first end of the capillary corresponds to or is smaller than the dimension of a single target cell, and the second end is so dimensioned to be smaller than the dimension of a single target cell to prevent a target cell received by the first end to pass through.

For example, a 1ml volume of fluid sample may be partitioned into 1 million cell extraction modules each containing a single 1 nanolitre droplet.

The micropore capillary may be a microchannel . The cell-receiving side may equivalently be terms a fluid-receiving side and it may receive a fluid sample that carries the target cell. While not essential, preferably, each of the plurality of cell extraction modules may comprise a micropore capillary as defined above.

The single layer array may be arranged over the surface of a planar disc, optionally having a surface with a hydrophilic configuration, such as by being configured in the Wenzel pattern.

In some embodiments, at least one micropore capillary may be configured with the first end wider than the second end. For example, the micropore capillary may be shaped like a funnel.

In preferred embodiments, the dimension of the first end of the capillary may be so dimensioned to be smaller than the dimension of a single target cell. Part of the target cell may be forced into the first end, thereby deforming and trapping the cell, and blocking the first end of the capillary. The first end may, e.g., be dimensioned based on the size and/or shape of the one or more target cells. The dimension of the first end may depend on the dimension of the target cell, but will typically be at least 0.1, 0.2, 0.3, 0.4, 0.5, 0.6, 0.7, 0.8, 0.9, 1, 1.5, 2, 2.5, 3, 3.5, or 4 µm and no more than 15, 14, 13, 12, 11 or 10 µm, e.g. 0.1-5 or 0.1-2 µm, e.g. about 1 µm.

In some embodiments, at least a portion of the micropore capillaries may each have identically dimensioned first ends. In some embodiments, a first portion of the micropore capillaries may, e.g., have a first end of a first dimension based on the size of the one or more target cells of a first predetermined cell type, and a second portion of the micropore capillaries may have a first end of a second different dimension based on the size of the one or more target cells of a second predetermined cell type.

The second end may, e.g., be so dimensioned to allow fluid to pass through (without allowing target cells to pass through). It may, e.g., be so dimensioned to allow the fluid to form droplets. The second end may, e.g., also be so dimensioned to allow non-target cells, i.e. cells that it is desired to dispose of, to pass through (without allowing target cells to pass through).

For example, the second end may have a diameter of about or less than 8, ,7, 6, 5, 4, 3, 2, 1 or 0.5 µm.

In preferred embodiments, the first end of at least one micropore capillary is formed into a well in fluid communication with the micropore capillary wherein the opening of the or each well is wider than the first end of the micropore capillary. For example, a cell extraction module may be formed as a well with an opening in the centre of its base that leads to a micropore capillary.

In some embodiments, the well is dimensioned to be at least 2, 3, 4, 5, 6, 7, 8, 9 or 10 times larger than the target cell. For example, the well may have a diameter of about 50-150 µm, e.g. 80-120 or about 100 µm; and/or a depth of about 50-150 µm, e.g. 80-120 or about 100 µm.

In some embodiments, where more than one micropore capillaries are each formed into a well, at least a portion of the plurality of wells may each be dimensioned based on the size and/or shape of the one or more target cells. By "dimensioned based on the size and/or shape of the one or more target cells" is meant that the well is dimensioned to capture and preferably retain an individual target cell. For example, a well may be dimensioned to be the same size as a target cell such that the well may physically retain the target cell, or a well may be dimensioned to be bigger than a target cell to accommodate the target cell while the target cell is retained by the corresponding micropore capillary. In such an embodiment the well dimension will depend on the dimension of the target cell but may be any other suitable and desirable size, for example be at least 2, 3, 4, 5, 6, 7, 8, 9, 10 or 6 µm in diameter and no more than 15, 14, 13, 12, 11 or 10 µm, e.g. 8-10 µm.

The plurality of wells may, e.g., be of the same dimension based on the size and/or shape of the one or more target cells of one predetermined cell type. Alternatively, a first portion of the plurality of wells may, e.g., be of a first dimension based on the size of the one or more target cells of a first predetermined cell type, and a second portion of the plurality of wells may be of a second different dimension based on the size of the one or more target cells of a second predetermined cell type.

The plurality of cell extraction modules may comprise or consist of at least of about 1000, 10,000, 50,000, 100,000, 500,000, 1 million, 1.1, 1.2, 1.3, 1.4, 1.5 or 1.6 million cell extraction modules, or any suitable and desirable number of cell extraction modules. The cell extraction modules may be arranged into radial sectors, which in preferred embodiments are on the surface of a planar disc. Advantageously, where the surface has a hydrophilic configuration, e.g. a Wenzel configuration as discussed above, then the hydrophilic surface promotes increases the ease of fluid slippage over the surface and aids the equal partitioning of the fluid sample so that target cells are received at each radial section, and the likelihood of each cell extraction module receiving a target cell is increased. This may be particularly advantageous when the fluid sample is distributed over the surface of the cell extraction device e.g. by application of centrifugal force, then each radial section may receive droplets containing target cells that may reach the different sections at different levels of centrifugal force.

In some embodiments, the cell extraction device may be provided with at least two electrodes. The or each pair of electrodes may be disposed across the opening of a well and may be arranged to measure an electrical parameter of the fluid sample within the well. The electrodes may form a capacitor in some examples, and the cell extraction device may be arranged to measure capacitance of the electrodes. Alternatively or additionally, the electrodes may be arranged to measure the conductivity or impedance of a fluid sample within the well. The measured capacitance, conductivity or impedance may then be used to determine one or more characteristics of the fluid and/or captured cell, the level of fluid and/or if the well is occupied by a cell or not. The fringe field of each electrode may be sensitive to the level of the wetting of the vertical electrodes on the sides of the well and this can provide a way to measure the level of fluid and/or to determine if the well is occupied by a cell as a captured cell clogs the opening of the capillary /microchannel . In preferred embodiments, each cell extraction module is provided with at least two electrodes.

In some embodiments, the cell extraction device may be provided with at least one field effect transistor. The or each field effect transistor may be fabricated along, or disposed within a well, such as being fabricated inside of the well. The field effect transistor may be arranged to detect a change in potential, a change in temperature and/or a photoelectric event within the corresponding well. The detected changes in potential and/or temperature, and/or photoelectric events may e.g. be used for monitoring changes in the sample, particularly the captured cell and/or the fluid (e.g. the cell milieu) within a cell extraction module, such as for monitoring changes in the contents of the well and/or changes in temperature.

A field effect transistor may be provided to any suitable and desirable part of a well. However, in preferred embodiments, the field effect transistor of each well is integrally formed (or fabricated) as the base of the corresponding well, for example around the opening in the base of the well.

In some embodiments, the cell extraction device may be provided with at least one micro-antennae, or an array of micro-antennas. A group of cell extraction devices may be provided with at least one or an array of microsized fractal antennas. The micro-antennas may consist of silicon, i.e. they may be all silicon microsized antennas. The micro-antennas may be arranged to operate for near field regime at the broad radio frequency (RF) range for continuous monitoring of the captured cell(s) and/or of the fluidic environment within the well. The micro-antenna may be disposed alone or in tandem within a well and/or arranged at the fluid extraction side or fabricated monolithically.

Each micro-antenna may be governed by an integrated circuit (IC) chip with a variable AC frequency generator source that powers the antenna from one frequency to a second frequency representing the frequency range for the antenna (which may be from 10MHz to 63GHz), and a signal detector which is in communication with each of the antennas, the row/ column decoders choose a specific antenna in an array and collects the transmitted and the reflected signals between each of the antennas over all frequency range.

The or each micro-antenna may be disposed within a well and arranged to receive a signal from the corresponding pair of electrodes and/or the corresponding field effect transistor. For example, the micro-antennae may be arranged to communicate with a communication module for continuous monitoring of the captured cells, fluid and well environment. Alternatively, the or each micro-antenna may be configured to function as one or more sensors that obtain data relating to the fluid sample within a well. The thus obtained data may be collected using suitable means and analysed.

One or more cell extraction modules may, e.g., be labelled with identification tags such as holographic tags. For example, the or each of the plurality of cell extraction modules may be labelled with a unique identification tag fabricated along its side, which may e.g. be read by a suitable and desirable reading or scanning device.

The plurality of cell extraction modules may, e.g. be evenly spaced. The distance between adjacent cell extraction modules may, e.g. be at least 10, 20, 30, 40, 50 µm or any suitable and desirable distance. The plurality of cell extraction modules may, e.g. be in the Wenzel pattern. Thus, in example embodiments the openings for the cell extraction modules (i.e. the microcapillary/microchannel openings) may create a Wenzel pattern over the surface of the cell extraction device, such as over the planar surface of a disc as discussed above.

In some embodiments, at least one of the plurality of cell extraction modules may, e.g. be provided with an affinity ligand, which may be configured to form a target-cell-adhesive coating. The affinity ligands may typically be synthetic, short macromolecules that meme the native ligands. The affinity ligand may, e.g., be tethered to a surface of the cell extraction module such that the ligand is configured to interact with its target on a target cell, such as a target macromolecule that is held on the surface of the target cell. The affinity ligand may, e.g., be arranged to form a monolayer covering at least a portion of the cell extraction module. Thus, in some embodiments, a cell-adhesive coating may be provided on an inner surface of one or more wells. The cell-adhesive coating may advantageously help to capture and retain a target cell within the well. The cell-adhesive coating may be provided on any part of the inner surface of a well. However, in preferred embodiments, the cell-adhesive coating is provided in the vicinity of the opening at the first end of the or each micropore capillary, e.g. as an annular ring around the opening.

Thus, when a cell is captured in a well, e.g. at the first end opening of a micropore capillary, if the cell membrane bears one or more target molecules, the one or more target molecules may bind specifically to one or more of the affinity ligands provided on the well. Such affinity binding may help to capture a target cell, and/or to retain a target cell, e.g. during a rinse or wash step.

The interaction between a target molecule and one or more affinity ligands may be of high affinity binding, and/or it may be of low affinity binding. In the case of low affinity binding, high avidity may be achieved through the interaction of a target molecule, typically a single target molecule, with a plurality of affinity ligands. Thus, the affinity ligands may be arranged in a manner that allows and promotes a high avidity biding to one or more target molecules on a target cell. The arrangement may, e.g., be a high density of affinity ligands.

The cell extraction modules/wells that are provided with an affinity ligand may each be provided with the same affinity ligand or combination of affinity ligands. Alternatively, a first plurality of cell extraction modules/wells may be provided with a first type of affinity ligand or combination of affinity ligands; and a second plurality of cell extraction modules/wells may be provided with a second type of affinity ligand or combination of affinity ligands.

Different affinity ligands may be specific for the same target molecule (such as for different regions of the same molecule), for different target molecules on the same target cell, or for different target molecules on different target cells. Combinations of different affinity ligands specific for the same target molecule, and/or specific for different target molecules on the same target cell may be used. For example, a combination of different affinity ligands may be provided in each cell extraction module/well. This may enable the capture in a specific manner of cells which are in a transitory phase of evolution that is indicative of the progression of a disease.

In some embodiments, a first portion of cell extraction modules/wells may be provided with one or more different affinity ligands specific for a first target molecule, such as Epithelial cell adhesion molecule (EpCAM), whilst a second portion of cell extraction modules/wells may not be provided with any affinity ligands, or may be provided with affinity ligands specific for a second target molecule, such as one of the cluster differentiation (CD) markers, e.g. CD45. Yet further portions of the cell extraction modules/wells may be provided with yet further different affinity ligands specific for yet further markers.

The device may be formed of any material suitable for micro-fabrication methods to achieve highly resolvable patterns. In preferred embodiments, the device may be formed as a wafer of a material selected from, e.g. silicon, AlGaN /GaN, a photoresist (e.g.: SU8), or any other suitable and desirable semiconductor material, novel compound materials suitable for forming three dimensional patterns using 3D micro-printing, injection moulding , stamping, flexographic printing etc. The device may, e.g., be fabricated into a form of a disc or any other form suitable for micro-fabrication . In embodiments where the cell extraction device is formed of a semiconducting material, one or more cell extraction modules may be arranged to function as a microelectromechanical (MEMS) device.

In embodiments where a pair of electrodes is disposed across the opening of more than one wells, the electrodes of each wells may be electrically connected through conducting wires, crisscrossing the surface of the cell extraction device/wafer and electrically connected to electrical pads arranged at the outer boundary of the cell extraction device/wafer, thus forming a two-dimensional matrix. The capacitance of each cell module can be assessed by the cartesian readings of the outer pads.

In another aspect, there is provided a cell analysis system comprising: a cell extraction device as defined herein; and a rotation device coupled to the cell extraction device configured to rotate the cell extraction device at a predetermined angular speed such that a centrifugal force is exerted on the fluid sample placed on the cell extraction device. In preferred embodiments, the cell extraction device may be set to rotate on a substantially horizontal plane. The cell extraction device may be a planar device, such as having the form of a planar disc. In some embodiments, the cell extraction device may be set to rotate on a plane at a predetermined angle (e.g. >0° and <90) relative to the axis of rotation. The cell extraction device may be in form of a cylinder set to rotate round its axis. This cylinder may be placed along with sample fluid in another cylinder of a bigger diameter with the suspension thus held between the two cylinders. In that arrangement the outer cylinder can act to spread the suspension over the face of the inner cylinder during the rotation.

Advantageously, as the fluid sample placed on the centre of the cell extraction device experiences the centrifugal force exerted thereon as a result of the cell extraction device being rotated by the rotation device, the fluid sample is distributed over the surface of the cell extraction device. As the fluid sample spreads over the surface of the cell extraction device, the fluid sample is partitioned into one or more of the plurality of cell extraction modules/wells, each of which receives a portion, e.g. a nanolitre droplet, of the fluid sample, while a cell in the droplet may be retained by the corresponding micropore capillary.

The cell analysis system may, e.g., further comprise a suction device, such as a pump, coupled to the cell extraction device and configured to provide suction through the micropore capillary of one or more cell extraction modules, e.g. to draw nanodroplets of the sample fluid to the inside of the one or more cell extraction modules. In doing so, a cell in a nanodroplet inside a cell extraction module may be retained by the corresponding micropore capillary opening as a result of the suction.

The cell analysis system may, e.g., further comprise a substrate for receiving the fluid from the fluid sample as it is being evacuated from the fluid-evacuating side, particularly nanoliter droplets of fluid. The substrate may, e.g., be provided (e.g. surface coated) with an affinity ligand, which may be configured to form a target-molecule-adhesive region. The affinity ligand may, e.g., be tethered to the surface of the substrate such that the ligand is configured to interact with its target on a target molecule. The affinity ligand may, e.g., be arranged on the substrate to form a monolayer as a pattern that corresponds to the pattern of the patterned surface, e.g. as a pattern of dots that may correspond to the pattern of the outlets from the wells.

The affinity ligand provided in the well and/or on the substrate may be a synthetic ligand selected from a group comprising peptides, peptidomimetics, aptamers, polymers that carry a ligand that attaches with a target or native macromolecules such as antibodies, or a combination thereof. Suitable peptides may, e.g., be 10-20 amino acids in length, e.g. about 15; suitable peptidomimetics may be of equivalent length.

The cell analysis system may, e.g., further comprise a sensor module configured to obtain data relating to the one or more target cells extracted by the cell extraction module.

For example, in embodiments where at least one of the plurality of cell extraction modules is labelled with a unique identification tag, the sensor module may, e.g., comprise a scanner configured to read the unique identification tag of the or each cell extraction module. The scanner may be provided by a camera such as a multispectral camera that can obtain image data related to the morphology of the cell while also scanning the tag of the or each cell extraction module, which may be a holographic tag as discussed above. As each tag can be identified and its content monitored, then this allows each cell extraction module, when configured to retain a singular cell of a particular identity, to act as a cell counting and identification module. The system then advantageously has the function of a cellular quantitative and qualitative device, which may be configured for analyzing a sample of a suspension of biological origin e.g.: a bodily fluid such as blood.

The sensor module may, e.g., comprise an environment monitoring system configured to continuously or periodically obtain data on the temperature, humidity, and/or the level of gases such as CO₂. This may relate to data for one or more cell extraction modules, e.g. to monitor the environment of the cell extraction modules. Moreover, the sensor module may, e.g., further comprise an environmental control system configured to control one or more of the temperature, humidity, and/or gas (e.g. CO₂) level of one or more wells. Thus, according to preferred embodiments, a cell extraction module/well may be uniquely identified, monitored and controlled.

The cell analysis system may, e.g., further comprise an incubation module configured to culture a target cell captured in a cell extraction module. The incubation module may, e.g., comprise one or more microfluidic channels each provided to one of the plurality of cell extraction modules. These channels may provide a means to replenish the fluid volume lost passively via the microchannel and to supplement the necessary ingredients for each one of the plurality of cell extraction modules. This may be accomplished with a fluid dispensing unit controlled via the output of sensors of each well, for example by use of one or more of the capacitor sensor, the field effect transistor and/or the micro-antenna discussed above. The fluid dispensing module may be configured to maintain optimal incubating conditions necessary for the cell maintenance and/or to elicit a cell stage (e.g.: proliferation, apoptosis etc.). Thus, according to preferred embodiments, cells captured by the plurality of cell extraction modules may be incubated in situ. Together with the sensor module, captured cells may be uniquely identified and monitored, nutrients may be provided to the captured cells and the environment may be carefully controlled via the incubation module, such as to optimise cell maintenance and growth and/or to induce a biochemical pathway or cellular stage.

The cell extraction device may be cleaned of any organic matter and/or sterilised after each use so as to be reused. The cell extraction device may therefore be regarded as recyclable. The cell extraction device may be removed from the cell analysis system and be cleaned using conventional means, such as in a conventional sterilisation unit. However, in some embodiments, the cell analysis system may, e.g., further comprise a sterilisation module configured to sterilise the cell extraction device in situ, thus enabling the sterilisation to be automated and further improving the efficiency of the cell analysis system.

In another aspect there is provided a method of cell extraction from a fluid sample using a cell analysis system provided herein.

The fluid sample may, e.g., be a body fluid or liquid biopsy, e.g., blood or a blood-derived product. Thus, the method may, e.g., be used to extract individual cells and/or serum derived vesicles and macromolecules from blood.

Thus, there is provided a method of cell extraction using a cell analysis system provided herein comprising applying a fluid sample onto the cell extraction device; activating the rotation device to rotate the cell extraction device at a predetermined angular speed such that a centrifugal force is exerted on the fluid sample applied to the cell extraction device (a 'centrifugation' step).

The method may, e.g., further comprise extracting fluid from the fluid sample from the plurality of wells by means of suction, e.g. operating at a transverse vector, which may be transverse to the plane of a planar form of the device, using a pump coupled to the cell extraction device configured to provide suction through one or more wells (a 'suction' step). According to embodiments, the suction exerts a force on the sample fluid as it approaches a well, urging a droplet of the sample fluid to enter the well and moreover exerts a force on the incoming target cells and/or on the sample fluid as it approaches a well, which may act to draw a droplet of the sample fluid into the well, thus further improving the ability of the cell extraction device to extract and retain target cells.

As mentioned above, the cell extraction modules/wells may be provided with an affinity ligand, which may be configured to form a target-cell-adhesive coating. The centrifugal force and suction may cause a cell to be captured (randomly) into a cell extraction module/well that does not contain an affinity ligand specific for that cell. To increase cell capture specificity, the method may include several rounds of cell capture. Thus, the method may further include a step of releasing and/or washing the non-specifically bound cells from the cell extraction modules/wells, which may be achieved by reducing or discontinuing the suction and/or applying a (washing) solution to the patterned surface (the fluid receiving surface of the cell extraction device) to dislodge the cells from the cell extraction modules/wells (a 'release' step). The release step may be performed so as to allow cells that are not specifically bound to affinity ligands to be released from their cell extraction modules/wells, whilst favouring the retention of cells that are specifically bound to affinity ligands.

The release step may be followed by a further centrifugation and/or suction step, which may provide a second sorting step using the centrifugation and/or suction forces. The method may comprise several rounds of (a) cell capture via centrifugation and/or suction steps followed by (b) a release and/or washing step. Performing several rounds of release and capture should increase the proportion of target cells that are captured in cell extraction modules/wells containing affinity ligands specific for said target cells.

As each round may be quick, performing several rounds allows the method to be highly efficient yet speedy.

As mentioned above, a cell extraction module/well may be provided with one or more different affinity ligands, which may each be specific for a different target molecule. The affinity ligands may be patterned into a monolayer, which may, e.g. be a few microns in width. For example, each different affinity ligand may be patterned into a monolayer, such that each monolayer is specific for a different target molecule.

This may allow the capture of target cells expressing at least one of the target molecules. For example, if the target cells are cells undergoing a transition from a first to a second stage, e.g. cancerous cells undergoing Epithelial-Mesenchymal Transition (EMT), a cell extraction module/well may be provided with an affinity ligand for a marker of the first stage, such as an epithelial marker, and with an affinity ligand for a marker of the second stage, such as a mesenchymal marker. Thus, the method may be used to extract, and optionally analyse, cells undergoing a transition from a first to a second stage, e.g. cancerous cells undergoing EMT.

The method may be used to extract and identify diseased cells, e.g. CTCs, so the method may be a method of early detection and/or diagnosis, or may form part of a method of early detection and/or diagnosis.

The method may, e.g., further comprise analysing the one or more target cells extracted by the cell extraction module. The analysis may involve using a sensor module.

The method may, e.g., further comprise incubating the target cell within the well under a predetermined condition. This may involve culturing the cell, which may comprise providing the cell with nutrients via a microfluidic system, such as a system comprising a dispensing system controlled by the sensors that monitor the cell and its milieu.

The method may, e.g., further comprise monitoring continuously or periodically the environmental condition of one or more wells comprising, e.g., one or more of the temperature, humidity, nutrient and/or gas (e.g. CO₂) level, and adjusting the monitored environmental condition based on a reference condition.

The method may, e.g., further comprise analysing the effect of a test compound on the cell, which may comprise contacting the cell with a test compound, e.g. via a microfluidic system/dispensing system, and analysing the cellular response to the test compound. For example, cytotoxicity of a test compound may be analysed by analysing the effect of the compound on cell viability, which may, e.g. conveniently be determined by using a viability stain.

The method may, e.g., further comprise analysing the ability of a test compound to bind specifically to the test cell, which may comprise contacting the cell with a test compound, e.g. via a microfluidic system, and analysing the binding of the test compound to the cell. For example, the test compound may be labelled with a detectable label such as a chromophore and the presence of the label on the cells may be determined.

As mentioned above, the cell analysis system may comprise a substrate for receiving fluid from the second end of the capillary, i.e. the outlet of the microchannel at the fluid extracting side. This substrate may be provided with affinity ligands. The method may, e.g., further comprise analysing the fluid received on the substrate, e.g. captured by affinity ligands. For example, membranous organelles e.g.: exosomes and/or macromolecules such as antibodies, clotting factors, albumin, carbohydrates, proteins, lipids, lipoproteins, chylomicrons, circulating tumour-DNA, circulating free DNA, and/or ribonucleoprotein complexes may be analysed.

Various embodiments of the present disclosure will now be described, by way of example only, and with reference to the accompanying drawings in which:
Fig. 1 shows an example of a disc according to an embodiment;
Fig. 2 shows a die of the disc of Fig. 1;
Fig. 3 shows an isometric view of the cell extraction modules/wells of a disc according to an embodiment;
Fig. 4 shows an exemplary case in which a cell is captured in a cell extraction module/well according to an embodiment;
Fig. 5 shows a top view of a single well according to an embodiment;
Fig. 6 shows a flow pipe used in a cell capturing system according to an embodiment;
Fig. 7 shows an optical lens used for disc monitoring according to an embodiment;
Fig. 8 shows schematically an optical system for monitoring a disc according to an embodiment; and
Fig. 9 shows a flow diagram of a method of body fluid processing according to an embodiment.

The present disclosure provides a device and a system for capturing cells, particularly rare cell, in a biological fluid sample, e.g. a blood sample. Embodiments of the device comprise a patterned surface, e.g. an array of cell extraction modules/wells, at least one cell extraction module/well being configured to capture a cell, which may e.g. be a target cell of a predetermined shape and/or size.

Herein, "rare cells" may include any cells that are present at a low concentration and/or as a low proportion of the total cells (overall cell population) in a biological fluid sample.

For example, rare cells may be present at a concentration of less than 1000, 900, 800, 700, 600, 500, 400, 300, 200, 100, 50, 40, 30, 20, 10, 9, 8, 7, 6, 5, 4, 3, 2, or 1 cells per milliliter of the biological sample. Alternatively or in addition, rare cells may be present as a very low proportion of the total cells in, e.g. at a ratio of rare cells to total cells of 1: ≥1 million, 1: ≥5 million, 1: ≥1 billion or 1: ≥5 billion.

The rare cell may, for example, be selected from blastomeres, circulating tumor cells (CTCs), stem cells, astrocytes, fetal cells, e.g. fetal nucleated red blood cells (fNRBCs), and microorganisms, such as bacteria.

The biological fluid sample may be a body fluid or a "liquid biopsy" sample, e.g. blood or a blood-derived fluid, e.g. blood that has been subjected to one or more processing steps, e.g. as discussed below in the 'enrichment' section. Any reference herein to "blood" should be understood to encompass suitable blood-derived fluids.

According to various embodiments, it is possible to isolate cells at an improved rate from complex bodily fluids by capturing an individual cell in each cell extraction module/well. In some embodiments, efficiency may be further improved by including a cell-type specific adhesion process to the system, in which process one or more cell extraction module/well is configured to be adhesive, e.g. selectively adhesive, to a predetermined cell type. It may then be possible to identify a captured cell by the cell-type specific layer to which it adheres, optionally in addition to the cell's morphological and/or biochemical characteristics and/or marker expression profile.

In some embodiments, the system may further comprise means to allow the cell to be contacted with a solution of interest, e.g. a nutrient solution to feed the cells, or a test solution to test the interaction between a test substance and the cell. This may e.g. include a microfluidics or microinjectors.

In some embodiments, the system may further comprise a suitable monitoring system which may comprise e.g. a laser reader and a sensor module (e.g. an ADC unit) such that each well may be identified with a unique holographic tag, readable by the laser reader, provided by each well, and monitored individually using sensor module. Alternatively or additionally the system may comprise a multispectral camera for imaging each of the wells and for identifying the wells via the holographic tag.

Embodiments of the cell capturing system comprise a device that comprises an array of cell extraction modules/wells on a planar disc. The surface of the disc may be provided with a hydrophilic configuration, for example by being roughed, such as the Wenzel configuration, in order to promote the wetting of the entire surface of the disc, along with slippage free motion of the fluid with its suspended particles across the surface. As mentioned above the Wenzel configuration may be provided (in full or in part) by the openings into the cell extraction modules, i.e. the openings into the wells. This then promotes more even partitioning of the particles (e.g. cells) in the sample fluid into the wells. During the first stage, a biological fluid sample is placed centrally on the device, which is in the form of a circular disc, then the disc is set to rotate. As the rotating disc is a non-inertial frame of reference the pseudo forces that act on a mass as it accelerates are the centrifugal and Coriolis forces opposed by the Euler force. The last two forces will cancel each other once the disc maintains a constant velocity. The biological fluid sample is hence spread over the rotating disc by centrifugal force whereby the cells of the biological fluid sample are separated and the cells are segregated into individual wells or compartments of the disc. One or more of the wells may be arranged to have a negative pressure gradient to provide the well with suction, e.g. through the base of the well (at the outlet opening in the base), to facilitate fluid entry into a well and to assist in the capture of a cell at the capillary opening of the well to further improve the efficiency of cell separation and allow cell binding to occur.

In some embodiments, the device is formed in the shape of a disc, and the disc may be formed of a semiconductor e.g. a silicon wafer, AlGaN /GaN, a photoresist (e.g. SU8), or any other materials suitable for 3D printing, such as ABS plastic, PLA, polyamide (nylon), glass filled polyamide, stereolithography materials (epoxy resins), silver, titanium, steel, ceramics, wax, photopolymers and polycarbonate, etc. In preferred embodiments, semiconducting materials are used to enable a monolithic micro electric mechanical (MEMS) device to be fabricated. A top surface of the disc comprises a two dimensional array of cell extraction modules/wells or compartments arranged into a Wenzel pattern for promoting superhydrophilicity. In some embodiments, the disc surface may be roughened into the Cassie Baxter pattern, thus some sectors may be hydrophilic such as via the Wenzel pattern, and others may guide the flow via hydrophobic configuration, such as via a Cassie Baxter pattern. In some embodiments, the plurality of wells may be identical in diameter, depth and shape. In some embodiments, the wells may differ in shape and/or size. In preferred embodiments, each well is substantially round (including polygonal), but other shapes are contemplated. Where the device is dedicated for the extraction of cells then the size of each well is selected to be sufficiently big to accommodate precisely or at least one target cell. Thus, the size of each well may correspond to the size of a target cell, or may be larger or much larger than the size of a target cell, and can be proportionately larger as the size of a target cell increases. In some embodiments, each well may be provided with a faucet or outlet in the form of a capillary that extends the thickness of the disc to allow fluid to drain down from the top surface of the disc through the base of the disc, where a container may, e.g., be provided to collect the fluid with the aid of suction applied to the outlet of each well, such as by applying suction to the fluid evacuating surface and hence applying a negative pressure to the fluid receiving surface via the inlets to the wells.

In some embodiments, the device is configured to capture target cells based on their physical properties. Rare cells are biomechanically distinct from certain other cells, particularly blood cells, in terms of size, density and/or elasticity, and they are generally larger than other blood components. In some embodiments, the dimensions of the cell extraction modules and/or wells may be selected to accommodate only a single target cell of a predetermined type, for example circulating tumor cells. In some embodiments, the extraction modules and/or wells may differ in shape and/or size for capturing cells of different types. In some embodiments, the extraction modules/wells may be of the same shape and/or size, while the diameter of the faucet opening diffs for capturing cells of different types while allowing the passage of unwanted (i.e. non-target) cells and/or the passage of fluid through the capillary.

Embodiments of the cell capturing system provide high-throughput analyses of rare cells directly from a biological fluid such as (whole) blood. In preferred embodiments, the system comprises three functional modules including:
(i) a cell selection module comprising a disc of an ultra-high aspect ratio, e.g. 200mm over 725µm, the disc is formed into a honeycomb or an array of approximately 1.6M wells, each well with a conduit (e.g. a microchannel) that filters or drains the fluid gathered in the well out through the base of the well;
(ii) a life sustaining module for maintaining the cell in each well, provided individually to one or more well or collectively to all wells; and
(iii) an imaging module for label-free cell counting (e.g. CTC counting) and imaging for phenotypic identification, morphological study, migration tracking, and optionally
(iv) a capacitor module for monitoring the liquid height in each well and/or a transistor for measuring the electric field at the well, such as in order to track biochemical changes.

In some embodiments, one or more cell extraction module/well of the disc may be coated with affinity ligands that specifically and selectively bind to the target cell.

In some embodiments the fluid that is filtered out of the well, which may inter alia include cell-derived vesicles, is collected and further processed. The fluid may, e.g., be cell-free fluid and/or may, e.g. contain cell-derived vesicles such as exosomes and/or macromolesules. The fluid that is filtered out of the well may include cell free DNA, circulating tumor DNA, cell free proteins, tumour cell free proteins, and/or membranous cell-derived vesicles such as exosomes. As the fluid moves through the substrates, which are layered underneath the fluid extraction surface, they filter the analytes according to size and retain them with affinity ligands tethered to the matrix of the substrate.

The affinity capturing ligand may, e.g. be a chemical molecule, peptide, peptidomimetic, aptamer or antibody, such as one that targets an analyte. The substrate layers can be removed separated one from the other and be further processed.

In preferred embodiments, the disc is formed from a solid state wafer e.g. a semiconductor such as silicon wafer, AlGaN /GaN, or a photoresist (e.g. SU8), but other suitable material may be used if desired. The disc may be formed with an upper layer for capturing cells and filtering out fluid from the sample, and a lower layer with a pixel structure where each pixel matches each well and provided with an individual sensing element (e.g., CHEMFET, patch antennae for permittivity measurements, optoelectronic detectors, electrodes). A transceiver layer may be provided at the base of the wafer for collecting data. Thus, the first module (disc or cell extraction device) may be considered as an array of microelectromechanical devices, signaled through e.g. a photoelectrical sensing system as individual pixels, each pixel representing a well, enabling the continuous spatial and temporal monitoring of the cells within the wells.

The upper layer of the disc may have an upper surface fabricated into a repeating pattern (e.g. Wenzel) that allows the complete wetting of the disc surface by the fluid when applied to with a centrifugal force, along with spreading of the fluid into a two dimensional interface over the entire surface and wall slippage of the fluid particles across the disc when the pattern is the Wenzel pattern. Distribution of the fluid is assisted by rheological effects such as shear-thinning intermolecular forces, a diffusive interface including suction fluxes culminating in the partitioning of the fluid into droplets which are pinned to the surface and solutes sorted randomly across all droplets.

A unit of the repeating pattern can be shaped into a well, which can be for partitioning the interface of fluid into a droplet as it meets the well and the capturing of a single cell, as well as filtering out the remaining fluid from the sample by means of the inlet and outlet of the well forming a microchannel that transverses the disc. The microchannel has an inlet at the bottom of the well , and an outlet the other surface of the fluid extraction side lower layer, with a pixel structure where each pixel matches each well and provided with an individual sensing element (e.g., ChemFET, patch antennae for permittivity measurements, optoelectronic detectors, electrodes, etc.). Multiple electrical transducing sensors can be embedded in each unit , externally or fabricated monolithically, such as: capacitor, field effect transistor and/or all silicon metamaterial antenna.

In preferred embodiments, the disc is provided with a plurality of electrodes. Each pair of electrodes is disposed across the opening of a well and arranged to measure the capacitance, conductivity or impedance of a medium within the well. In addition to measuring electrical parameters of the medium within a well, it has been contemplated that the electrodes may be used, by applying an AC potential at a predetermined frequency, to electrically focus (or direct) a target cell towards the centre of a well. The predetermined frequency may be selected based on its effectiveness for directing and confining a particular cell type to a chosen location. Moreover, it has been contemplated that a predetermined potential may be applied across one or more pair of electrodes to dynamically change the size of cells and/or to influence cell entry, thus changing the types of cells that can be captured. It has been further contemplated that the electrodes may be used to study the electrophysiological properties of a cell by studying the change in AC dielectrophoretic characteristics of an incubating cell and its migration as a function of frequency, by applying an AC potential to one or more pairs of electrodes at varying frequency.

Moreover, the disc may be provided with a plurality of field effect transistors. Each field effect transistor is disposed within a well and arranged to detect a change in potential (e.g. of the fluid at the microchannel), a change in temperature and/or a photoelectric event within the corresponding well. In preferred embodiments, the field effect transistor of each well is integrally formed or fabricated so that its top gate or its channel serves as the base of the corresponding well. In preferred embodiments, the disc is provided with a plurality of micro-antennae. Each micro-antenna is disposed within a well and arranged to receive a signal from the corresponding pair of electrodes and/or the corresponding field effect transistor. The micro-antennae may be arranged to communicate with a communication module for continuous monitoring of the captured cells and well environment. Alternatively, one or more micro-antennae may be configured to function as one or more sensors that obtain data relating to the fluid sample within a well. The thus obtained data may be collected using suitable means and analysed.

In preferred embodiments, the disc is provided with a plurality of micro-sized antennae. The micro-antennae may, for example, be one or more micro-sized fractal antennae or all silicon metamaterial antenna. Each a pair of micro -sized antennas is disposed within the walls of a well of the cell extraction module within a well and arranged underneath the disc. The micro-sized antennae may be arranged to communicate one with each other across a well or when arranged side by side in a distance that allow them to sense their fringe field, wherein each antenna operates at the near field regime across the radio frequency, microwave, subTHz and THz frequency ranges, emitting and receiving the scattered and the transmitted EM spectra emitted by the molecules in response to the irradiation, and read by a network analyser (scalar or vector), that analyzes the power density distribution of the received radiation at the ports of the antennae (e.g.: the s11, s12, s21 s22 coefficients).

The EM spectra emitted by the molecules in response to the irradiation are analysed to obtain the power distribution at the ports of the antennae, and one or more scattering coefficients across a frequency range are determined. This allows the permittivity of the fluid sample to be studied, and moreover allows the transmitted EM frequencies that are specific to a particular solute or vesicles to be identified, and monitored.

A pair of micro-antennae may be arranged in tandem and set in a communicating mode with each other with the well sample therebetween, for measuring at the near field frequencies the scattering coefficients (the S parameters) when irradiating the fluid sample at a RF and/or microwave radiation. The micro-antennae may, for example, be one or more micro-sized patch antennae. One or more micro-sized patch antennae may be arranged to measure the permittivity of a sample within a well (cell extraction module) and to follow its changes over time. One or more micro-sized patch antennae may be arranged to operate at the near-field regime at radio frequency (RF), microwave, subTHz and THz frequency ranges. A pair of micro-sized patch antennae may be disposed within the wall of a well (cell extraction module) and arranged in tandem opposite each other to receive a signal within a predetermined range of wavelength, and transmit to a processor for a vector network analysis that analyses the power density distribution of the received radiation (e.g.: the S11, S12, S21 S22 coefficient). The micro-sized patch antennae may be arranged to communicate with each other, such that, while the biological sample is within the wells, the well fluid and captured cells are continuously monitored.

According to preferred embodiments, since the disc is formed of a semiconducting material, it is possible to arrange each well to function as a microelectromechanical (MEMS) device. In embodiments where a pair of electrodes is disposed across the opening of each well and/or at opposite walls, the electrodes of all wells may be electrically connected through conducting wires, crisscrossing the surface of the cell extraction device/wafer and electrically connected to electrical pads arranged at the outer boundary of the cell extraction device/wafer, thus forming a two-dimensional matrix of capacitors that is capable of detecting the emptying or filling of the wells.

Preferably, the first module (disc) is configured such that it is replaceable, and can be readily integrated with conventional laboratory devices such as spin coater (for rotating the disc), hot plate, oven, cell incubator, multispectral camera (e.g. as discussed above), and/or laser reader (similar to a laser reader in a CD player) to allow continuous morphological observation. Thus forming a modular cell analysis system.

Embodiments of the (rare) cell capturing system provide an Automatic Cell Analyzer (ACA) for parallel processing of rare cells, e.g. circulating tumor cells. The system benefits from being modular, each module may be provided in the same or different locations, and the system does not require all modules to be present to achieve the advantageous effects of the present invention. The disc of the first functional module may provide up to 2,000,000 wells in conjunction with a parallel processing and monitoring module. The first module may be fabricated using conventional fabrication technologies. According to preferred embodiments, each well is able to culture a single cell and may be labeled by micro-hologram on the external side of each well for identification.

In some embodiments, the system may additionally be provided with the following features: temperature control; gas supply and control; micro-fluid technology; optical cell ID; electromagnetic motors for disc rotation; a replaceable evacuation chamber coupled to the base of the disc; and/or auxiliary monitoring units including near-field solid state micro-sized antennae, ring oscillators, Raman spectroscopy analyzer, fluorescent analyzer, R-DNA analyzer, temperature, humidity, flow velocity, flow pressure, gas pressure, gas sensors, etc.

In further embodiments, the system may be provided with automatic sterilization units, for example using sterilization fluids, UV, O₂, and/or high temperature processing. After each sterilization, the system may be used for repeat analyses for different biological fluid samples.

The disc may be regarded as comprising two sides - a cell-receiving side (top side) and a fluid-evacuating side (bottom side). Each well compartment is provided with a micropore capillary that extends the thickness of the disc. On the cell-receiving side of a well, the capillary is shaped like a goblet that is wider at the opening; on the fluid-evacuating side, the capillary narrows into a pore-like outlet to allow fluid to form a meniscus. A suitable substrate, e.g. a filter paper, saturated and/or surface modified with one or more suitable affinity ligands, may be provided on the fluid-evacuating side below the plurality of capillaries to receive the droplets formed at each pore-like outlet. Preferably, the substrate is placed at such a distance from the capillary outlet that the fluid meniscus of each droplet contacts the surface of the substrate as it forms. Preferably, the one or more ligands may be provided on the substrate (filter paper) in a pattern or array that replicates or corresponds to the pattern or array formed by the capillaries. Alternatively, the one or more ligands may be applied evenly throughout the substrate. The one or more ligands may be applied with a ligand per layer of substrate with multiple layers arrayed one on top under the other, with the layers of the substrate of same material but of different surface modification. It has been further contemplated that the substrate (filter paper) may instead be laid on the top surface/cell-receiving side of the disc with the positions of the ligands matching the pattern of the wells.

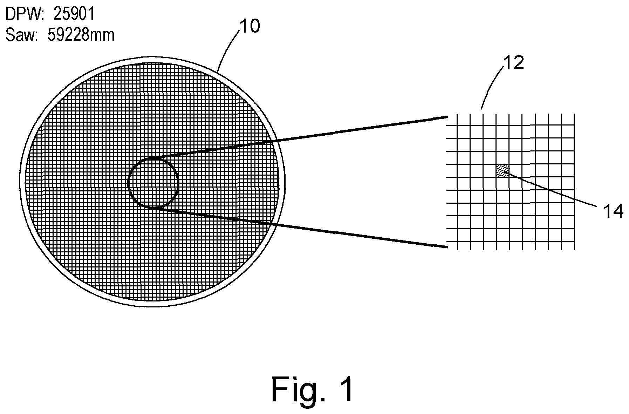

Figs. 1 to 6 show views of a wafer disc (e.g. quartz) for use in the first module according to an embodiment of the system. The disc contains about 1.65 million compartments or wells, each capable of capturing and housing a single cell. In particular, one such disc may contain 25900 dies, each die of 1mm², with each die containing 64 compartments or wells that house a single cell, totaling 1.65M. Each cell has a diameter of 100µm and a depth of 100µm. The wafer serves as a filter in a flow system including liquid reservoir with biological sample for analysis.

At the bottom of each well in the center there is provided a faucet or outlet with a diameter ranging from 2µm to 10 µm (depending on the disc) leading to a micropore capillary. The faucet/outlet is configured to allow the passage of fluid and cells with diameters below the diameter of the faucet/outlet, aided by a suction force (if applied) applied through the corresponding micropore capillary. When a cell of a diameter greater than the diameter of the faucet/outlet enters the well, it blocks the corresponding faucet/outlet and thereby ceases further liquid flow into the faucet/outlet. Thus, only one cell of a predetermined size (size of the faucet/outlet) or above can be captured within the well. The single captured cell can give rise to a single colony-forming unit (CFU) culture inside the well, thus facilitating unambiguous cell identification.
Fig. 1 shows an embodiment of a disc 10 used for the first functional module of the system. In the present embodiment, the disc 10 is 20cm in diameter formed of an array of rectangular chips or dies 12 each of 1mm². Each die 12 comprises an array of wells 14, where each well 14 has a diameter of 100µm and a depth of 100µm. The disc 10 comprises about 25,900 dies.

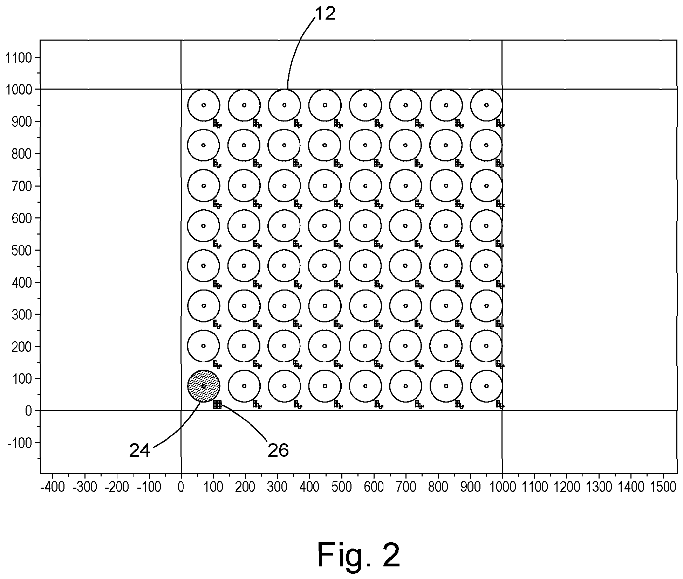

Fig. 2 shows an enlarged view of a die 12 of the disc 10. In the embodiment, each square millimetre die 12 of the disc 10 comprises 64 analyser wells 24. According to the embodiment, each well 24 is labelled with an optical tag 26 that can be used to uniquely identify each well 24.

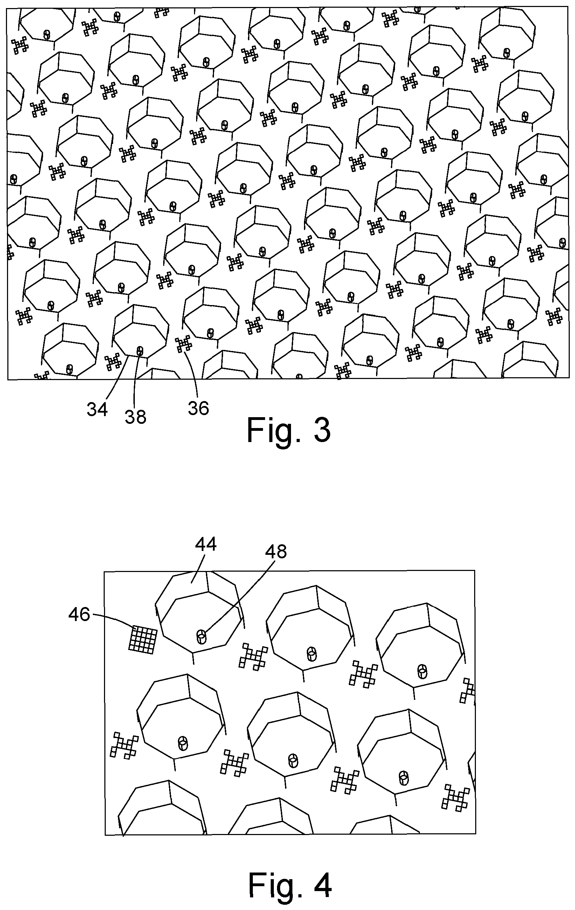

Fig. 3 shows an isometric view of a plurality of wells 34. Each well 34 is again labelled with a corresponding optical tag 36. According to the embodiment, each well 34 is provided with a central faucet 38.
Fig. 4 illustrates an exemplary case when a biological cell is captured in a well 44 labelled with an optical tag 46. The faucet 48 of the well 44 is blocked by the captured cell and further flow through the well 44 is not possible. Thus, according to the embodiment, each individual well is configured to only capture a single cell.

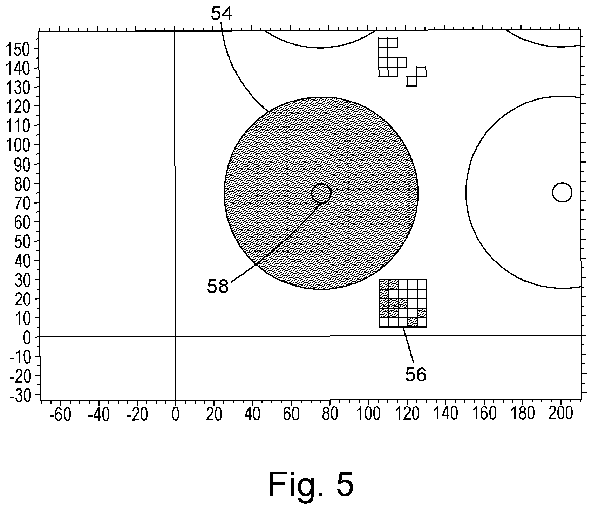

Fig. 5 shows a top view of a single well 54 with a diameter of 100µm and a corresponding tag identifier 56. In the centre of the well 54 there is provided a faucet 58 with a diameter of 5µm that allows liquid to flow out of the well 54. The well 54 is configured to capture a single biological cell with a diameter corresponding to the size of the faucet 58. For much larger or smaller cells, e.g. microorganisms such as bacteria, a disc provided with respectively larger or smaller faucet diameter may be used.

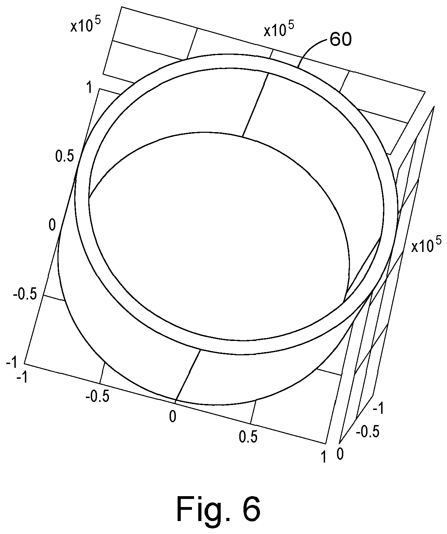

Fig. 6 shows a flow pipe 60 with a diameter of 20cm hermetically connected to the disc frame for draining fluid out of the wells.

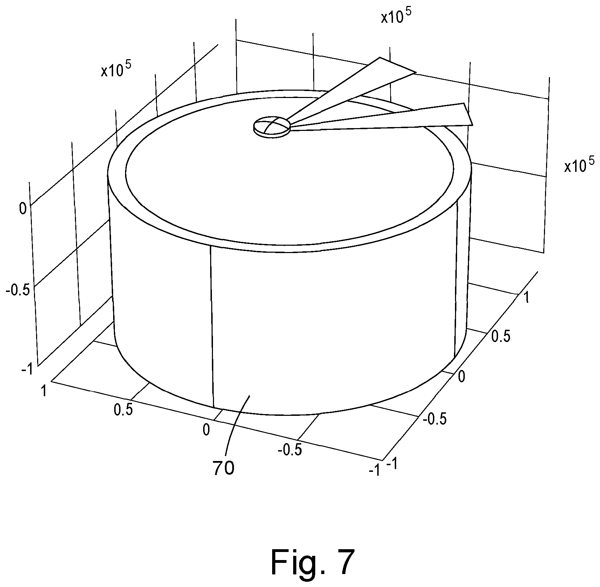

Fig. 7 shows an optical lens 70 with motor drivers that can be used in an optical monitoring system for monitoring the wells on a disc. The disc is rotated about its axis and may be read by a suitable sensor such as a laser reader (similar to a CD player).

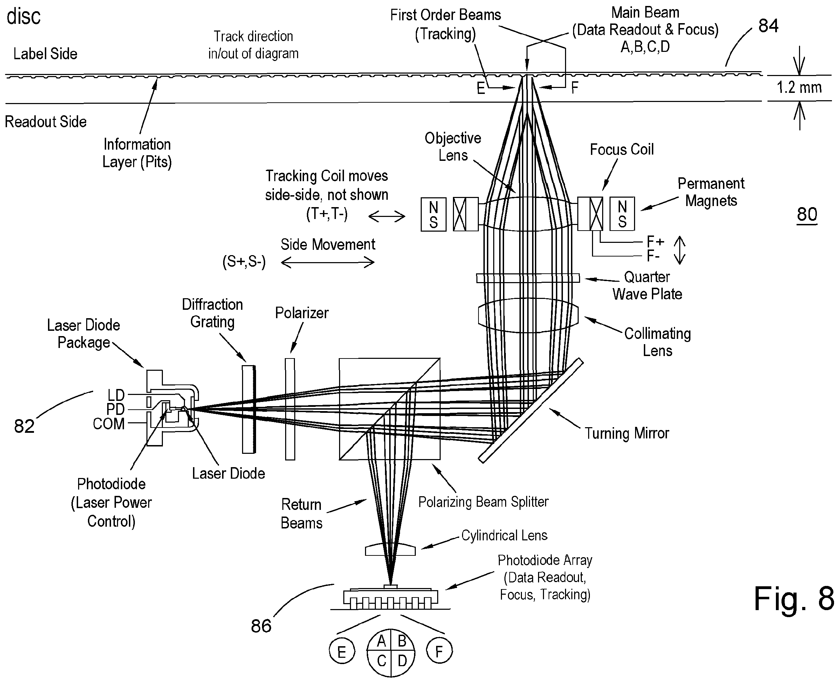

Fig. 8 schematically shows an optical sensor system 80 that can be used for monitoring the wells on a disc 84. A laser diode package 82 is provided to generate a reference laser beam, which is directed to a readout side of the disc 84. The reference beam is directed to each well and the diffracted beam is directed towards a photodiode array 86 for data readout. The optical sensor system 80 may be used to read an optical (e.g. holographic) identification provided to each well, such as the optical tag 36 for well 34.

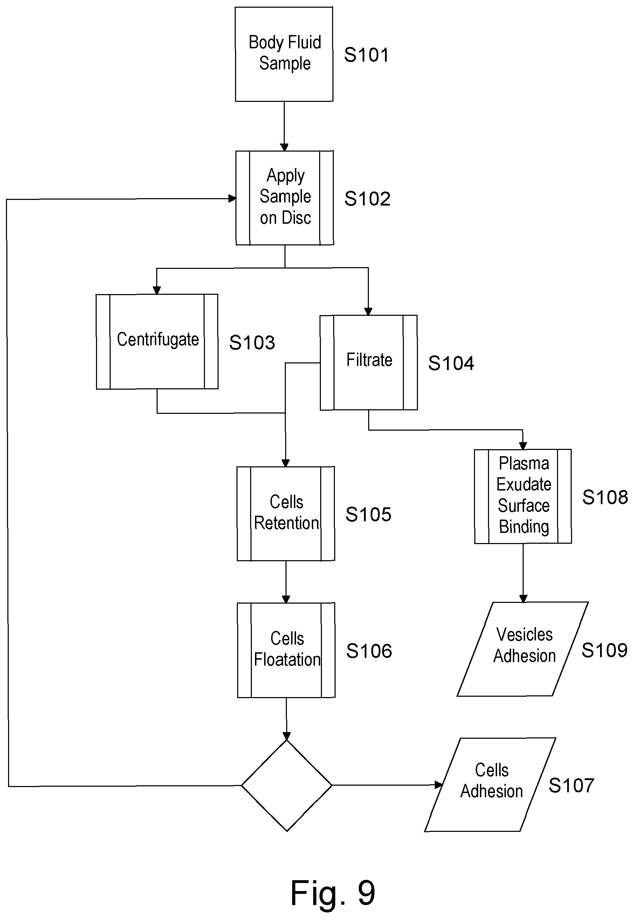

Fig. 9 shows a flow diagram of a method of body fluid processing performed by a cell capturing system according to an embodiment. The method comprises first obtaining a body fluid sample, such as a blood sample (step 101), which may be a provided sample, so the method need not include an active step of removing a sample from the human or animal body. The sample, e.g. of a few ml volume, is applied to the center of a disc such as the disc 10 (step 102). The disc can be regarded as having two sides - a cell-receiving (top) side and a fluid-evacuating (bottom) side.

The disc is set to rotate at a predetermined angular speed and the sample is separated under the centrifugal force caused by the rotation (step 103), and the fluid from the sample is distributed, preferably evenly, over the surface. The fluid sample is thus partitioned into plural droplets, with each droplet being urged into each well by the application of a suction force through the bottom surface or base of the wells (step 104). The disc may be placed on a shaft of a spin coater. The shaft is then set to rotate at a low angular speed (e.g. 20 rpm for 30 sec, ramping time of 30 sec) to disperse the sample evenly on the cell-receiving side of the disc. A vacuum may be activated so that the disc is pushed against the rotating shaft. Preferably, suction is provided, by a suction device such as a pump coupled to the disc, to each well through each corresponding micropore capillary. While the rotation of the disc generates a centrifugal force that distributes the fluid sample across the disc, the suction aids the evacuation of fluid from the sample from the wells.

The target cells are individually retained in each well (step 105). In some embodiments, capture of cells into wells is repeated two or more times, which may involve a release step of dispersing the cells back into solution , e.g. using a suitable solvent ("cell floatation") (step 106) and repeating step 103. The use of solvent overcomes the small range binding forces between the cells and the wells, and the cell floatation step removes the cells that are no longer retained in the wells when suction ceases, e.g. the pump is stopped. Using cell specific adhesive coating, a cell is adhered to a respective well if recognition ensues(step 107). During one or more of the retention steps (step 105) and/or cell floatation step (step 106), a substrate saturated with one or more affinity ligands, e.g. filter paper, may be laid on top of the disc to allow other target cells to bind with corresponding ligands. The affinity ligands may be provided in a pattern or array that replicates the patterned surface of cell extraction modules/wells on the cell-receiving side of the disc.

According to the embodiment, each well is tapered off into a capillary that extends the entire thickness of the disc. The capillary is shaped such that it exerts maximum resistance to the passage of a predetermined target cell while exerting minimal resistance to the passage of fluid. Thus the opening of each capillary on each side of the disc is preferably of a different shape. The shape of the capillaries is preferably determined by the material the disc is made of. For example, when a silicon substrate is used, a pore-like opening is formed. The precise form and shape of the capillaries are determined by their function. On the cell-receiving side, the capillary opening is a goblet shape to accommodate an incoming cell, and on the fluid-evacuating side the capillary opening is pore-like to allow the buildup of a fluid meniscus, when the liquid buildup is caused by inertial impaction, gravity or Brownian diffusion. When fluid movement is driven by electrodynamics, hydrodynamics or both, the shape of the capillary opening may be determined empirically.

The flow filtrate (e.g. plasma, which may e.g. contain vesicles) passing through the capillary at step 104 may be captured on a substrate, e.g. filter paper. The capillary opening may be configured to allow the filtrate to form nanodrops. The filter paper may contain affinity ligands (step 108), which may be in an array that replicates the pattern of cell extraction modules/wells on the cell-receiving side of the disc. Components such as vesicles present in the filtrate may be selectively captured on the substrate using suitable affinity ligands (step 109).

The disc may be made reusable. In this case, the disc may then be sterilized using suitable method and a new sample may be applied to the disc for processing.

The system and method may in some embodiments be used to test various properties of captured target cells. For example, the target cells may be contacted with testing solutions comprising one or more test substances to assay the response of the cells to the test substance(s). This may allow e.g. the testing of the susceptibility or resistance of target cells to test drugs; and/or the affinity or specificity of test affinity ligands.

Embodiments of the system and method thus provide a single array that comprises over 1 million cell extraction modules/wells, each capable of accommodating a single cell entity supported by a branched microfluidic system or a microinjection system, which may be a dispensing system, that directs and/or supplies the inflow of the testing solutions to the interrogated subpopulations of cells at a specific sector of the array and collects the outflow. The microinjection system may be provided as a retractable module of a patterned array of pipettes configured and arranged for simultaneous fluid extraction and/or fluid refilling of each well, each pipette being provided to extract fluid from and refill the fluid for a corresponding well. Alternatively or additionally the well may empty of fluid passively via flow through the microchannel outlet. The extraction/refilling may be operated through a pressure or a vacuum pump regulated to extract fluid from or feed fluid into each well through the corresponding pipette/a tube. The fluid to be inserted into the wells may be provided in a replaceable multicompartment cartridge, each compartment being in fluid communication with a pipette. The volume of fluid may be measured, such as via the capacitor discussed above, in order to determine the volume of fluid needed for replenishing the fluid in the well.

The microfluidic system or the injection system may be monitored and controlled via a sensor system, which measures and collects data from each well relating to the physical, biochemical and morphological characteristics of the cell. The collected data indicates the cellular events that the cells go through. The sensor system may be configured to measure and monitor the temperature, humidity and gas (e.g. pCO₂) level within each well such that cell growth medium can be optimized accordingly.

In use, the disc is first secured on a spin coat through a substrate such as a wet filter paper placed underneath and secured by applying vacuum temporarily. Then a suitable volume, e.g. 20ml of biological fluid sample for a 200mm diameter disc is placed at the center of the disc. The disc is then ramped up e.g. for 30 sec, set to rotate at an angular speed e.g. of 20 rpm for 60 sec, and them ramped down e.g. for 30 sec. The flotation-washing step is done by repeating the spin coat step with flotation buffer. e.g. :five times with the same volume of phosphate saline buffer, e.g. at pH=7.45, I=155mM. The disc may then be removed and allowed to incubate under humid conditions e.g. at 10°C for 5 min.

The physical process described above can be summed as revolving three dimensionally a fluidic suspension over a planar surface with suction, extending the Bodewadt /Einstein model of 2-dimensional viscous fluid flow. Their model maintains that the no slip condition at the solid & smooth surface creates a viscous boundary layer that reduces the circumferential velocity component in the vicinity of the surface and reduces the radially directed centripetal acceleration. Mass conservation gives rise to a spiralling upward flow of oscillatory nature with the particles velocity is radially directed inwards and increases in magnitude from zeRo at the bottom all the way to the surface with air.

The oscillation can be damped by partial slip and with sufficient suction velocity through the planar surface, the axial flow is directed in the downwards direction instead of upwards. The Bodewadt boundary layer becomes substantially thinner and the oscillatory behavior vanishes.

A slippage free surface assisted with suction is predicted to eliminate the oscillations completely by abolishing reason for the existence of a radial vector and promoting fluid spreading by thinning the Boudewadt boundary layer. Since blood is a non-Brownian and Stokerian suspension then its particles are expected to be carried with the fluid and be uniformly distributed with it.

Total slippage is achieved by roughening the surface especially a surface that contains periodically distributed regions of zero surface shear stress. The surface can be roughened into a regular Wenzel pattern with a repeating unit made of the well of the cell extracting module.

For a disc with a radius *r_{b}*, when a blood sample is placed centrally on the disc, the spread of the blood sample from its initial central position radially away to the circumference of the disc, when the disc is at rest, is determined by the critical radius for cell adhesion according to the equation: $τ = 3 Qη / {πrkh}^{2}$ (1) where *τ* is the shear stress, Q is the flow rate of the blood, *η* is the viscosity of the blood, r is the radius of the boundary up to which the blood has spread, and h is the height of the blood sample. It can be seen that the shear stress *τ* is highest at the plate epicenter and proportionately decreases with the radius r.

When the disc is set to rotate with an angular velocity *Ω*, a tangential velocity, *W =* Ω*r*, is imparted to the blood that is in contact with the disc, together with a centrifugal force that induces a radial outflow of blood from the center of the disc towards the outer perimeter of the disc. The entrainment of a fluid axially into a boundary layer and the exit of the fluid radially through the boundary layer is determined by the "free-disc entrainment rate". It is also determined by the local rotational Reynolds number (based on the tangential velocity of the disc and the radius from the disc axis) with the surface roughness of the disc affecting the boundary layer and the flow may become turbulent or stay laminar.

The surface roughness parameters are determined by the geometry of a unit well, including well size and shape and the mesoscopic distribution of the wells on the disc surface. The wetting is further promoted by the negative pressure exerted normal to the plane of the rotating disc that causes a transverse flow of the fluid into a well and out through the faucet provided at the base of each well.

In example embodiments the disc has a surface that is roughened in accordance with the Wenzel pattern. The Wenzel parameters are determined by the periodiocity of the unit well and the geometry of a unit well, which are the well size and shape.

The wells of the disc are so shaped and sized to reduce the translational forces of shear and centrifugal force that act on the blood and act in conjunction with the electrostatic Poisson Boltzmann state function of the sticky coat at the bottom of the wells, to promote the dynamics of the cells settling into each well and reaching equilibrium. Thus, . Two forces act together: a long range and a short range force. Experiments have demonstrated that the surface of the disc extends a force at long range that is not restricted to specific molecular contact and is dependent on the morphological shape of receiving surface.

Conventional technologies require an additional pre-separation step ("enrichment step") during which high volumes of body fluids are processed so that a sufficient number of cells can be recovered for a subsequent chemical separation step. Commonly, the volume which is gathered is vastly larger than the volume that the system can process. This restriction leads to the requirement for a step of sample concentration, performed for example by density sedimentation. Auxiliary physical separation steps are based on size, shape, compressibility, deformability or dielectric property parameters, where a range of methods may be used, for example micro filters inertia, lateral displacement, electrophoresis, acoustophoresis, affinity chromatography, magnetophoresis, dielectrophoresis, or combinations thereof. After the enrichment step, cells are then separated and sorted. Consequently, cell capturing using conventional methods is time-consuming and inefficient. Conveniently, the method of the invention does not require such an enrichment step, i.e. it may be carried out on a sample that has not been subjected to such an enrichment step, although a sample that has been subjected to such an enrichment step may be used, if desired.

Techniques that rely solely on cell adhesion to the surface are similarly time-consuming.

The adhesion of a cell onto a surface is determined by physicochemical properties of substrates such as the surface free energy, the surface polarity, the presence of functional groups and surface charges.

Adhesion of a cell onto a surface is a two-stage event comprising first of a fast stage of "jumping-into-contact" followed by a slower process of reaching dynamic equilibrium at the cellular level, where the cell gradually changes its shape and establishes contact and balance between adhesion and elastic properties. The shell of the cell dictates the equilibrium while the fluid contents dictate the rate of approach to equilibrium.

The "jumping-into-contact" stage is determined by the surface mesoscopic structure such as the dimension and shape of the cell extraction module/well, and is dependent on the state function (Poisson-Boltzmann-Debye) at the cell extraction module/well surface dictated by the surface potential dictated by its physical chemistry attributes of shape and surface chemistry..

Embodiments of the present invention expedite the jumping-into-contact stage through centrifugal force by disc rotation and/or application of suction through the base of each cell extraction module/well on the incoming cell. The suction or pressure gradient is formed by a faucet of a few microns located at the center of each well that extends through the thickness of the disc. This faucet, or outlet, can be the outlet of the microchannel as discussed above.

In the kinetic phase of the equilibrium stage, the adhesive forces exerted by the adhesive molecules (affinity ligands) that bind the cell shell with the cell extraction module/well is balanced with the elastic forces that keep the cell spherical. Such forces may push the cell away. In addition, there may be competitive binding of contaminating molecules. Once bound, it may take a long time (e.g. several minutes) for the bound cell to change from a spheroid shape and adjust its membrane into a stable shape that conforms to the topography of the cell-adhesive layer. To understand the mechanism of the equilibrium stage, the cell may be modeled as a spherical viscoelastic shell containing viscous fluid. The physical parameters that govern the response time of the deformation include the area compressibility, shear modulus and viscosity of the fluid. The geometry of the contact zone affects the kinetics of binding. It is speeded up once it is retained on the surface by applying suction that deforms the membrane and expose it to the affinity binding monolayer. Consequently, more of the cell's outer membrane is in touch with the monolayer and the better the adhesion becomes and the faster is the equilibrium reached (within minutes).

The small size of a cell (typical diameter 1-10µm), the low magnitude of the adhesion force (0.1-100nN) with the length of time it takes for the cell to establish proper docking, inhibit the efficiency of the conventional separation process and consequently increase the costs of the current cell-separation process.

Embodiments of the present system offers a quick and efficient separation process that can be performed economically. The kinetic to equilibrium phase is expedited by the application of drag (suction) forces normal to the disc that operates on the cells. The cells in the biological sample can be regarded as rough spheroids moving in a fluid with a Reynolds number of ~300, towards a hole located at the epicenter at the base of each well where suction is experienced by the fluid by the application of a negative pressure gradient from the surface of the disc through the respective capillaries (microchannels) towards the fluid-evacuating side of the disc. Consequently, the cell is pulled towards the hole (capillary/microchannel inlet) and is eventually captured or "locked" at the hole entrance, which is configured to be too narrow to allow the cell to pass through.

It may be advantageous to promote the deformation of the cell membrane to facilitate binding. Thus, in some embodiments, the material of the cell extraction module/well may be selected to have a surface free energy, surface polarity, functional groups, and/or surface charges optimised to facilitate the second stage of the cell adhesion. In some embodiments, the second stage may be enhanced by providing the cell extraction module/well with ligands, e.g. affinity ligands that may form a cell-adhesive coating. Thus, the cells may bind onto a cell-adhesive coat that retains the incoming cells with an adhesion energy higher than *kT,* where *k* is the Boltzmann constant and T is the temperature of the fluid measured in Kelvin.

The affinity ligand may be specifically selected to target a macromolecule, for example a glycoprotein, which is present at the cell surface. Cell type identifying information may be gained when the macromolecule is cell-type specific. In the embodiments such binding involves the formation of a monovalent bond between a small molecule, e.g. an affinity ligand, and its binding site on the macromolecule. Since a cell may comprise multiple macromolecules of the same or similar type, the cell-adhesive coating may be provided with a plurality of affinity ligands to achieve multiple bonds.

The establishment of contact and subsequent deformation between a substrate and a free-form cell in suspension depends on the chemical contact formed locally where the cell membrane meets the surface of the substrate. Usually this contact area is dominated by polymer layers on both sides. Specific binding can form between e.g. the glycoprotein of the outer membrane of a cell (e.g., cell adhesion molecules CAM) and the surface polymers on the substrate (e.g., Lectins and immunoglobulin). Multiple bonds formed between the cell and the surface polymers, eventually tethering the cell to and flattening it against the surface of the substrate. However, a conventional method that does not include the features of the cell extraction device provided herein is poorly controlled and time-consuming.

Moreover, conventional platforms that separate CTCs in blood focus mainly on carcinomas, as evident by their usage of antibodies that are specific to the epithelial cell membrane proteins (e.g. EpCAM or different cytokeratin in the Cell Search^{®} system). However, there is a high probability that these epithelial cell biomarkers are lost during the epithelial-mesenchymal transition that the carcinoma cancer cells undergo, as the cells acquire higher motility that allows them to escape into the blood stream from the primary tumor from which they originated. Cells in transit will flag both epithelial and mesenchymal biomarkers. Therefore, there is a need for a better type specific binding of the target cell using more than a single binder for a cell displaying heterogenous membrane proteins.

It is therefore desirable to provide an improved restrictive binding of the target cell e.g. via recognizing through binding a plurality of cell membrane proteins. It is therefore contemplated that a plurality of affinity ligands may be used, each for a different cell membrane protein, where each affinity ligands may be of low affinity towards its target while the cumulative binding exerts a sufficiently high affinity on a cell.

According to present embodiments, these capabilities may be attained by means of synthetic biology, namely synthetic peptides or aptamers that can be construed as to have any affinity towards a target, also a low one when needed. Being of small size and rod like shape enable their assembly into a monolayer of high directionality and high packing order with no steric hindrance

According to present embodiments, in order to keep the cell deformed a plurality of affinity ligands are provided, each binding specifically with a macromolecule which is present on the cell surface, such that the cell may be docked and

According to present embodiments, in order to promote the deformation of the cell membrane and/or to keep the cell deformed, a plurality of affinity ligands are provided, each binding specifically with a macromolecule which is present on the cell surface, such that the cell may be docked and tethered to the substrate surface quickly. Moreover, through the use of multiple affinity ligands, additional cell-type identifying information may be obtained when the cell-type specific macromolecule of a captured cell are identified through analyzing the affinity ligands on the surface of a given well. In the embodiments such binding involves the formation of a monovalent bond or multivalent bonds between a small molecule, e.g. a ligand, and its binding site on the macromolecule of a cell or more than one ligand and their binding sites on the same macromolecule of a cell. Since a cell may comprise multiple macromolecules of the same or similar type, the adhesive coating of each well is preferably provided with as many ligands as possible to achieve multiple bonds.

Conventionally, antibodies are used to bind target cells. However, forming a monolayer on a substrate with antibodies may be undesirable, as the bulkiness of antibodies may cause causes steric hindrance; and genetic hypervariability of antibodies leads to a wide variety of shapes. Moreover, the multiplicity of amino acids that can serve as linkers to the substrate surface may result in a disarrayed surface. Optimal binding is achieved when all binding events are equivalent and independent. Such binding may be achieved when the adhesive coating comprises a monolayer of affinity ligands arranged in a configuration that allows both maximal packing (10¹⁴ molecules/cm² or ~2 molecules/nm²), with maximal display are of binding sites. This combination may be achieved when the ligand having a two dimensional configuration, being wiry or rod like form. Their packing into a monolayer causes no steric hindrance between neighbouring ligands.

At one end, the 'wire' harbours a single moiety that docks it to the adhesive coating surface, i.e. the surface of the cell extraction module/well, while the other end carries the affinity site for binding with the macromolecule of the incoming cell.

Suitable ligands may include short peptides of 10-20, e.g. ~15 amino acid length, with a sequence that allow them a fully extended conformation form to have in a solution having physiological conditions possessing an end group that docks each peptide to their destined surface to form a highly oriented monolayer which is homogenous in its affinity towards a target carried on the membrane of the incoming cell. In some embodiments aptamers are used together with, or instead of peptides. The sequences of such affinity ligands may display biomimetic characteristics that imitate the binding domains of biological macromolecules such as antibodies, lectins, or other macromolecular structures.

Being synthetically designed, their chemical affinity towards a particular site can be reduced or enhanced by replacing one or more an amino acid in the sequence of the peptide (and/or by changing the order of the amino acids (or equivalent units) .

The homogeneity of the adhesive coating ensures that all cell extraction modules/wells that are surface modified by the same affinity ligand represent equivalent and independent binding sites.

Soft lithographic techniques such as microcontact printing (µCP), microfluidic printing (µFP) and laminar flow patterning (LFP) may be used to modify the surface of each well in a highly repetitive and industrious way.

The same peptides and apatamers will be used to surface modify the substrate that lines the fluid extraction surface underneath and its purpose is to receive the fluid extruded through the fluid extraction face. same methods of microcontact printing, microfluidic printing and laminar flow patterning it will be used to pattern the substrate with appropriate affinity ligands, filter paper is a suitable substrate.as such filters can be layered one upon the other , each layer catering for the specific capturing of a unique target , devoting its surface entirely to bind the necessary ligands each layer is surface modified in a pattern that conforms with the pattern formed by the outlets of the fluid extrusion modules over the surface of the fluid extraction face.

Layering the filters one upon the other aligning their ligand modification spots create a 3 dimensional chromatography tool that utilize both size exclusion and affinity binding for the simultaneous chromatography of millions of droplets driven through the stacked substrate layers.

Each layer can be subsequently separated, cut and manipulated for the purpose of farther molecular physico- biochemical assays.

Conventional techniques allow the surface of a single well to be modified with a single receptor. However, micro-contact printing may enable the surface of a well to be modified with more than one receptor by locating the receptors at different loci on the well's surface. Thus, it is possible for a well to sample multiple receptors of the same cell, increasing the probability of a cell to be captured.

Surface heterogeneity can assist in binding same macromolecule. Being structurally complex, a membrane protein may display several sites for a given ligand to bind with different binding constants. There are also cases in which the multiple binding becomes cooperative, positively or negatively. Embodiments disclosed herein enable control of the ligand concentration and fabricate coatings with a pre-conceived ligand concentration, and provide information about subtyping.

The CTCs derived from different types of tissues differ significantly from each other in size, shape, and immunophenotyping profile, while variation within CTCs derived from the same tissue of origin is broad across the morphological and immune-phenotypical dimensions. Therefore, accurate detection of CTCs based on morphological and immune-phenotypical profiling remains a challenge. Additionally, CTCs may be damaged and fragmented, *in vivo* and/or *in vitro,* due to multi-step cell preparation processes, causing inaccurate detection and misinterpretation. Most importantly, the CTCs may evolve, e.g. from carcinoma to mesenchymal type, and acquire additional macromolecules that may be carried together when in the transitory phase.

Heterogeneous surfaces may be provided to capture CTCs that co express and carry multiple surface markers, e.g., breast cancer CTC which is EpCAM negative with HER²⁺/EGFR⁺/HPSE⁺/Notch¹⁺ known to metastasize in the brain in mouse models, or CTC which are amidst an Epithelial-Mesenchymal Transition phase whereby tumor cells gradually make a transition from epithelial phenotype into a mesenchymal one during their metastatic progression, ostensibly via down regulation of epithelial markers (EpCAM, E-cadherin, cytokeratine, etc.) and upregulation of mesenchymal expression (e.g. vimentin) to achieve more invasive phenotype. It has been observed that mesenchymal proportion amongst a CTC population increases during chemotherapy treatment. Thus an association may be found between the epitopes and the clinical events.

Present embodiments allow enrichment CTCs to be targeted which escape detection with current EpCAM affinity based platforms. Although EpCAM has been shown to be an effective marker for CTC capture, it is limited in expression. Moreover, there are CTCs with no EpCAM that may be overlooked as in the case of CTCs with potential for creating brain metastasis, where a subgroup is found to be EpCAM negative in non-small cell lung cancer (NSCLC) patients.

Affinity ligands, such as peptides, aptamers, antibodies, according to present embodiments allow the creation of a well coating to be optimally packed while carrying more than a single ligand especially when using small ligands, with each ligand being configured to a different macromolecule, where the proportion between bound ligands is directly related to their mixing proportions and their interspacing throughout the coating is randomised. Improved binding of a single macromolecule can be achieved by the use of more than one single receptor, or using isoforms of the same peptide with different binding affinities towards the same molecule target.

The study of the captured cells may involve the study of cell morphology and morphological changes , migration , apoptosis and other events related to the phenotypic display of the CTC observed, allowing the analysis of cellular activities such as, spreading, migration, or biochemically through markers, proliferation, apoptosis, anoikis and the like.

Morphologically, the small size of the cells and the low magnitude of adhesion force (0.1-100nN) dictate the usage of a microscope to study each well separately and to study the relevant parameters to improve well occupation. Since each well is individually tagged, the system enables the remote sensing over time domain. A dispensing system system will supplement each well according to its loss in volume and nourish and supply each of the approximately 1.6 million wells individually will enable also the high throughput and large scale pharmaceutical testing. The automatic monitoring and automated assaying should reduce even eliminate labour costs. The use of monitors, e.g. a capacitor for one or more or each well enables continuous or periodic monitoring of the capturing process, as a cell that blocks the pore will affect the liquid level and the electric potential of the well milieu. The efficiency of the cell sorting and retaining can be determined as the number of wells that retain a cell is kept constant over time

Such a system that allows singular cell examination and monitoring over time while providing many permutations of environmental cues may realize the prospect of how to identify the subpopulations of rare cells that are not committed to one particular fate from start and study the mechanisms and realize the ability to direct them towards a selected fate by establishing a set of environmental cues and a set of mutations.

Any reference herein to "at least one" should be understood to encompass one, some or all as distinct possibilities, all of which are explicitly contemplated. Thus, e.g. a statement that "at least one" micropore capillary (microchannel) may be configured with the first end wider than the second end should be understood to mean that it is contemplated that one, some or all micropore capillaries may be configured in this way.

## Claims

1. A cell extraction device comprising:
a plurality of cell extraction modules (34, 44, 54) arranged in a single layer array, the single layer array having a cell-receiving side wherein an opening of at least one of the plurality of cell extraction modules on the cell-receiving surface is configured to receive and retain a single target cell from a fluid sample, and a fluid-evacuating side wherein an opening of the or each of the plurality of cell extraction modules on the fluid-evacuating side is configured to allow fluid from the fluid sample to be evacuated from the cell extraction device,
wherein at least one of the plurality of cell extraction modules comprises a micropore capillary (38, 48, 58) configured at a first end that is open on the cell-receiving side to receive a target cell, and configured at a second end that is open on the fluid-evacuating side to allow fluid to pass through, and
wherein at least one micropore capillary is configured with the first end wider than the second end, wherein the dimension of the first end of the capillary corresponds to or is smaller than the dimension of a single target cell, and the second end is so dimensioned to be smaller than the dimension of a single target cell to prevent a target cell received by the first end to pass through.

2. The cell extraction device of claim 1, wherein the single layer array is arranged over the surface of a planar disc (10, 84), and the surface is provided with a hydrophilic surface configuration, preferably, wherein the surface is roughened in the Wenzel pattern.

3. The cell extraction device of claim 2, wherein the disc is configured to be rotated to thereby distribute the fluid sample across the surface of the disc

4. The cell extraction device of any preceding claim, wherein each of the plurality of cell extraction modules (34, 44, 54) comprises a micropore capillary (38, 48, 58), wherein the first ends of a first portion of the plurality of micropore capillaries (38, 48, 58) are of a first dimension that corresponds to or is smaller than the dimension of a first target cell, and the first ends of a second portion of the plurality of micropore capillaries are of a second different dimension that corresponds to or is smaller than the dimension of a second different target cell.

5. The cell extraction device of any preceding claim, wherein the first end of at least one micropore capillary (38, 48, 58) is formed into a well (34, 44, 54) in fluid communication with the micropore capillary, wherein the opening of the or each well is wider than the first end of the micropore capillary, optionally wherein the one or more wells (34, 44, 54) are dimensioned based on the size and/or shape of the one or more target cells to capture and retain a target cell.

6. The cell extraction device of claim 5 wherein one or more affinity ligands are provided on an inner surface of one or more wells (34, 44, 54), wherein said affinity ligands are preferably arranged to form a cell-adhesive coating, e.g. as a monolayer, wherein the monolayer can be created as to coat the surface of the well directly or modify the surface of a structure, grown inside the well or inserted into the well to occupy its void whereby the structure can be of organic matter as hydrogel, carbon nano shells, inorganic matter such as silica microbeads or hybrid structures.

7. The cell extraction device of claim 6, wherein the affinity ligands are provided around the opening at the first end of each micropore capillary (38, 48, 58), e.g. as an annular ring.

8. The cell extraction device of claim 6 or 7, wherein
(a) the cell extraction modules/wells (34, 44, 54) that are provided with an affinity ligand are each provided with the same affinity ligand or combination of affinity ligands; or
(b) a first plurality of cell extraction modules/wells is provided with a first type of affinity ligand or combination of affinity ligands; and a second plurality of cell extraction modules/wells is provided with a second type of affinity ligand or combination of affinity ligands.

9. The cell extraction device of any one of claims 5 to 8 comprising at least two electrodes, wherein the or each pair of electrodes are disposed across the opening of a well (34, 44, 54) and arranged to measure the capacitance, conductivity or impedance of a sample within the well, wherein the sample comprises a cell and/or fluid, wherein the or each pair of electrodes are configured to apply an AC potential across the corresponding well to direct a target cell towards the centre of the well (34, 44, 54).

10. The cell extraction device of any preceding claim, wherein at least one of the plurality of cell extraction modules is labelled with a unique identification tag.

11. The cell extraction device of any preceding claim, wherein the device is formed in the shape of a disc with a material selected from a group comprising silicon, AlGaN /GaN, a photoresist, other semiconductor material, or a combination thereof, or novel compound materials suitable for forming three dimensional patterns using micro-printing, injection moulding, stamping, and/or flexographic printing.

12. A cell analysis system comprising:
a cell extraction device of any preceding claim; and
a rotating device coupled to the cell extraction device configured to rotate the cell extraction device at a predetermined angular speed such that a centrifugal force is exerted on the fluid sample placed on the cell extraction device.

13. The cell analysis system of claim 12, further comprising a substrate for receiving the fluid from the fluid sample evacuated from the fluid-evacuating side, the substrate being provided with one or more affinity ligands configured to capture one or more target molecules through affinity binding.

14. The cell analysis system of claim 12 or 13, further comprising a suction device coupled to the cell extraction device configured to generate suction through one or more of the plurality of cell extraction modules.

15. A method of cell extraction using a cell analysis system of any one of claims 12, 13 or 14 comprising:
applying a fluid sample onto the cell extraction device;
activating the rotation device to rotate the cell extraction device at a predetermined angular speed such that a centrifugal force is exerted on the fluid sample applied to the cell extraction device.

## Patentansprüche

1. Zellextraktionsvorrichtung, umfassend:
eine Vielzahl von Zellextraktionsmodulen (34, 44, 54), die in einem Array mit einer einzigen Schicht angeordnet sind, wobei das Array mit einer einzigen Schicht eine Zellaufnahmeseite aufweist, in der eine Öffnung mindestens eines der Vielzahl von Zellextraktionsmodulen an der Zellaufnahmeoberfläche konfiguriert ist, um eine einzige Zielzelle aus einer Fluidprobe aufzunehmen und festzuhalten, und eine Fluidabführseite, in der eine Öffnung des oder jedes der Vielzahl von Zellextraktionsmodulen auf der Fluidabführseite konfiguriert ist, um zu ermöglichen, Fluid aus der Fluidprobe aus der Zellextraktionsvorrichtung abzuführen,
wobei mindestens eines der Vielzahl von Zellextraktionsmodulen eine Mikroporenkapillare (38, 48, 58) umfasst, die an einem ersten Ende, das auf der Zellaufnahmeseite offen ist, konfiguriert ist, um eine Zielzelle aufzunehmen, und an einem zweiten Ende, das auf der Fluidabführseite offen ist, konfiguriert ist, um es Fluid zu ermöglichen, hindurchzutreten, und
wobei mindestens eine Mikroporenkapillare mit dem ersten Ende breiter als dem zweiten Ende konfiguriert ist, wobei die Abmessung des ersten Endes der Kapillare der Abmessung einer einzigen Zielzelle entspricht oder kleiner ist, und das zweite Ende so bemessen ist, um kleiner ist als die Abmessung einer einzigen Zielzelle zu sein, um zu verhindern, dass eine vom ersten Ende aufgenommene Zielzelle hindurchtritt.

2. Zellextraktionsvorrichtung nach Anspruch 1, wobei das Array mit einer einzigen Schicht über der Oberfläche einer ebenen Scheibe (10, 84) angeordnet ist und die Oberfläche mit einer hydrophilen Oberflächenkonfiguration bereitgestellt ist, wobei die Oberfläche vorzugsweise im Wenzel-Muster aufgeraut ist.

3. Zellextraktionsvorrichtung nach Anspruch 2, wobei die Scheibe konfiguriert ist, um gedreht zu werden, um dadurch die Fluidprobe über die Oberfläche der Scheibe zu verteilen.

4. Zellextraktionsvorrichtung nach einem vorstehenden Anspruch, wobei jedes der Vielzahl von Zellextraktionsmodulen (34, 44, 54) eine Mikroporenkapillare (38, 48, 58) umfasst, wobei die ersten Enden eines ersten Abschnitts der Vielzahl von Mikroporenkapillaren (38, 48, 58) eine erste Abmessung aufweisen, die der Abmessung einer ersten Zielzelle entspricht oder kleiner ist, und die ersten Enden eines zweiten Abschnitts der Vielzahl von Mikroporenkapillaren von einer zweiten, unterschiedlichen Abmessung sind, die der Abmessung einer zweiten, unterschiedlichen Zielzelle entspricht oder kleiner ist.

5. Zellextraktionsvorrichtung nach einem vorstehenden Anspruch, wobei das erste Ende mindestens einer Mikroporenkapillare (38, 48, 58) zu einer Vertiefung (34, 44, 54) gebildet ist, die mit der Mikroporenkapillare in strömungstechnischer Kommunikation steht, wobei die Öffnung der oder jeder Vertiefung breiter ist als das erste Ende der Mikroporenkapillare, wobei die eine oder die mehreren Vertiefungen (34, 44, 54) optional basierend auf der Größe und/oder Form der einen oder mehrerer Zielzellen bemessen sind, um eine Zielzelle einzufangen und festzuhalten.

6. Zellextraktionsvorrichtung nach Anspruch 5, wobei auf einer inneren Oberfläche einer oder mehrerer Vertiefungen (34, 44, 54) ein oder mehrere Affinitätsliganden bereitgestellt sind, wobei die Affinitätsliganden vorzugsweise angeordnet sind, um eine Zelladhäsionsbeschichtung, z. B. als Monoschicht, zu bilden, wobei die Monoschicht erzeugt werden kann, um die Oberfläche der Vertiefung direkt zu beschichten oder die Oberfläche einer Struktur, die im Inneren der Vertiefung gewachsen ist oder in die Vertiefung eingesetzt wird, um deren Hohlraum auszufüllen, zu modifizieren, wobei die Struktur aus organischem Material wie Hydrogel, Kohlenstoffnanoschalen, anorganischem Material wie etwa Siliziumdioxid-Mikrokügelchen oder Hybridstrukturen bestehen kann.

7. Zellextraktionsvorrichtung nach Anspruch 6, wobei die Affinitätsliganden um die Öffnung am ersten Ende jeder Mikroporenkapillare (38, 48, 58) herum, z. B. als kreisförmiger Ring bereitgestellt sind.

8. Zellextraktionsvorrichtung nach Anspruch 6 oder 7, wobei
(a) die Zellextraktionsmodule/Vertiefungen (34, 44, 54), die mit einem Affinitätsliganden bereitgestellt sind, jeweils mit dem gleichen Affinitätsliganden oder Kombination von Affinitätsliganden bereitgestellt sind; oder
(b) eine erste Vielzahl von Zellextraktionsmodulen/Vertiefungen mit einem ersten Typ eines Affinitätsliganden oder einer Kombination von Affinitätsliganden bereitgestellt ist; und eine zweite Vielzahl von Zellextraktionsmodulen/Vertiefungen mit einem zweiten Typ eines Affinitätsliganden oder einer Kombination von Affinitätsliganden bereitgestellt ist.

9. Zellextraktionsvorrichtung nach einem der Ansprüche 5 bis 8, die mindestens zwei Elektroden umfasst, wobei die oder jedes Paar Elektroden über der Öffnung einer Vertiefung (34, 44, 54) disponiert und angeordnet sind, um die Kapazität, Leitfähigkeit oder Impedanz einer Probe innerhalb der Vertiefung zu messen, wobei die Probe eine Zelle und/oder ein Fluid umfasst, wobei die oder jedes Paar Elektroden konfiguriert sind, um ein Wechselstrompotential über der entsprechenden Vertiefung anzulegen, um eine Zielzelle in Richtung der Mitte der Vertiefung (34, 44, 54) zu lenken.

10. Zellextraktionsvorrichtung nach einem vorstehenden Anspruch, wobei mindestens eines der Vielzahl von Zellextraktionsmodulen mit einem eindeutigen Identifikationsetikett beschriftet ist.

11. Zellextraktionsvorrichtung nach einem vorstehenden Anspruch, wobei die Vorrichtung in Form einer Scheibe mit einem Material, ausgewählt aus einer Gruppe, die Silizium, AlGaN/GaN, einen Fotolack, ein anderes Halbleitermaterial oder eine Kombination davon umfasst, oder neuartigen Verbundmaterialien gebildet ist, die zum Bilden von dreidimensionalen Mustern unter Verwendung von Mikrodruck, Spritzguss, Prägen und/oder Flexodruck geeignet sind.

12. Zellanalysesystem, umfassend:
eine Zellextraktionsvorrichtung nach einem vorstehenden Anspruch; und
eine mit der Zellextraktionsvorrichtung gekoppelte Drehvorrichtung, die konfiguriert ist, um die Zellextraktionsvorrichtung mit einer vorbestimmten Winkelgeschwindigkeit zu drehen, sodass auf die Fluidprobe, die auf der Zellextraktionsvorrichtung platziert ist, eine Zentrifugalkraft ausgeübt wird.

13. Zellanalysesystem nach Anspruch 12, das weiter ein Substrat zum Aufnehmen des Fluids aus der Fluidprobe, das aus der Fluidabführseite abgeführt wird, umfasst, wobei das Substrat mit einem oder mehreren Affinitätsliganden bereitgestellt ist, die konfiguriert sind, um ein oder mehrere Zielmoleküle durch Affinitätsbindung einzufangen.

14. Zellanalysesystem nach Anspruch 12 oder 13, das weiter eine mit der Zellextraktionsvorrichtung gekoppelte Saugvorrichtung umfasst, die konfiguriert ist, um einen Sog durch eines oder mehrere der Vielzahl von Zellextraktionsmodulen zu erzeugen.

15. Verfahren zur Zellextraktion unter Verwendung eines Zellanalysesystems nach einem der Ansprüche 12, 13 oder 14, umfassend:
Aufbringen einer Fluidprobe auf die Zellextraktionsvorrichtung;
Aktivieren der Drehvorrichtung, um die Zellextraktionsvorrichtung mit einer vorbestimmten Winkelgeschwindigkeit zu drehen, sodass auf die Fluidprobe, die auf der Zellextraktionsvorrichtung aufgebracht ist, eine Zentrifugalkraft ausgeübt wird.

## Revendications

1. Dispositif d'extraction de cellules, comprenant :
une pluralité de modules d'extraction de cellules (34, 44, 54) agencés en un réseau en couche unique, le réseau en couche unique présentant un côté réception de cellules dans lequel une ouverture d'au moins l'un de la pluralité de modules d'extraction de cellules sur la surface de réception de cellules est configurée pour recevoir et retenir une seule cellule cible d'un échantillon de fluide, et un côté évacuation de fluide dans lequel une ouverture du ou de chaque module de la pluralité de modules d'extraction de cellules sur le côté évacuation de fluide est configurée pour permettre au fluide de l'échantillon de fluide d'être évacué du dispositif d'extraction de cellules,
dans lequel au moins l'un de la pluralité de modules d'extraction de cellules comprend un capillaire micropore (38, 48, 58) configuré au niveau d'une première extrémité qui est ouverte du côté réception de cellules pour recevoir une cellule cible, et configuré au niveau d'une seconde extrémité qui est ouverte du côté évacuation de fluide pour permettre le passage du fluide, et
dans lequel au moins un capillaire micropore est configuré avec la première extrémité plus large que la seconde extrémité, dans lequel la dimension de la première extrémité du capillaire correspond, ou est inférieure, à la dimension d'une seule cellule cible, et la seconde extrémité est dimensionnée de manière à être inférieure à la dimension d'une seule cellule cible pour empêcher le passage d'une cellule cible reçue par la première extrémité.

2. Dispositif d'extraction de cellules selon la revendication 1, dans lequel le réseau en couche unique est agencé sur la surface d'un disque plan (10, 84), et la surface est dotée d'une configuration de surface hydrophile, de préférence, dans lequel la surface est rendue rugueuse selon le motif Wenzel.

3. Dispositif d'extraction de cellules selon la revendication 2, dans lequel le disque est configuré pour être mis en rotation afin de distribuer ainsi l'échantillon de fluide à travers la surface du disque.

4. Dispositif d'extraction de cellules selon une quelconque revendications précédente, dans lequel chacun de la pluralité de modules d'extraction de cellules (34, 44, 54) comprend un capillaire micropore (38, 48, 58), dans lequel les premières extrémités d'une première partie de la pluralité de capillaires micropores (38, 48, 58) sont d'une première dimension qui correspond, ou est inférieure, à la dimension d'une première cellule cible, et les premières extrémités d'une seconde partie de la pluralité de capillaires micropores sont d'une seconde dimension différente qui correspond, ou est inférieure, à la dimension d'une seconde cellule cible différente.

5. Dispositif d'extraction de cellules selon une quelconque revendication précédente, dans lequel la première extrémité d'au moins un capillaire micropore (38, 48, 58) forme un puits (34, 44, 54) en communication fluidique avec le capillaire micropore, dans lequel l'ouverture du ou de chaque puits est plus large que la première extrémité du capillaire micropore, facultativement dans lequel les un ou plusieurs puits (34, 44, 54) sont dimensionnés en fonction de la taille et/ou de la forme des une ou plusieurs cellules cibles pour capturer et retenir une cellule cible.

6. Dispositif d'extraction de cellules selon la revendication 5, dans lequel un ou plusieurs ligands d'affinité sont disposés sur une surface intérieure d'un ou plusieurs puits (34, 44, 54), dans lequel lesdits ligands d'affinité sont de préférence agencés pour former un revêtement d'adhésion cellulaire, par exemple sous forme de monocouche, dans lequel la monocouche peut être créée de manière à recouvrir directement la surface du puits ou à modifier la surface d'une structure, développée à l'intérieur du puits ou insérée dans le puits pour occuper son vide, selon lequel la structure peut être constituée de matière organique telle qu'un hydrogel, des nanocoques de carbone, de la matière inorganique telle que des microbilles de silice ou des structures hybrides.

7. Dispositif d'extraction de cellules selon la revendication 6, dans lequel les ligands d'affinité sont disposés autour de l'ouverture au niveau de la première extrémité de chaque capillaire micropore (38, 48, 58), par exemple sous forme d'anneau.

8. Dispositif d'extraction de cellules selon la revendication 6 ou 7, dans lequel
(a) les modules/puits d'extraction de cellules (34, 44, 54) qui sont munis d'un ligand d'affinité sont chacun munis du même ligand d'affinité ou de la même combinaison de ligands d'affinité ; ou
(b) une première pluralité de modules/puits d'extraction de cellules sont munis d'un premier type de ligand d'affinité ou de combinaison de ligands d'affinité ; et une seconde pluralité de modules/puits d'extraction de cellules sont munis d'un second type de ligand d'affinité ou de combinaison de ligands d'affinité.

9. Dispositif d'extraction de cellules selon l'une quelconque des revendications 5 à 8 comprenant au moins deux électrodes, dans lequel la ou chaque paire d'électrodes est disposée à travers l'ouverture d'un puits (34, 44, 54) et conçue pour mesurer la capacité, la conductivité ou l'impédance d'un échantillon dans le puits, dans lequel l'échantillon comprend une cellule et/ou un fluide, dans lequel la ou chaque paire d'électrodes est configurée pour appliquer un potentiel CA à travers le puits correspondant pour diriger une cellule cible vers le centre du puits (34, 44, 54).

10. Dispositif d'extraction de cellules selon une quelconque revendication précédente, dans lequel au moins l'un de la pluralité de modules d'extraction de cellules est étiqueté avec une étiquette d'identification unique.

11. Dispositif d'extraction de cellules selon une quelconque revendication précédente, dans lequel le dispositif se présente sous forme de disque avec un matériau choisi dans un groupe comprenant le silicium, l'AIGaN/GaN, une résine photosensible, un autre matériau semi-conducteur ou une combinaison de ceux-ci, ou de nouveaux matériaux composés appropriés pour former des motifs tridimensionnels par micro-impression, moulage par injection, estampage et/ou impression flexographique.

12. Système d'analyse cellulaire comprenant :
un dispositif d'extraction de cellules selon une quelconque revendication précédente ; et
un dispositif rotatif couplé au dispositif d'extraction de cellules configuré pour faire tourner le dispositif d'extraction de cellules à une vitesse angulaire prédéterminée de telle sorte qu'une force centrifuge est exercée sur l'échantillon de fluide placé sur le dispositif d'extraction de cellules.

13. Système d'analyse cellulaire selon la revendication 12, comprenant en outre un substrat pour recevoir le fluide provenant de l'échantillon de fluide évacué du côté évacuation de fluide, le substrat étant muni d'un ou plusieurs ligands d'affinité configurés pour capturer une ou plusieurs molécules cibles par liaison d'affinité.

14. Système d'analyse cellulaire selon la revendication 12 ou 13, comprenant en outre un dispositif d'aspiration couplé au dispositif d'extraction de cellules configuré pour générer une aspiration à travers un ou plusieurs de la pluralité de modules d'extraction de cellules.

15. Procédé d'extraction de cellules utilisant un système d'analyse cellulaire selon l'une quelconque des revendications 12, 13 ou 14, comprenant :
l'application d'un échantillon de fluide sur le dispositif d'extraction de cellules ;
l'activation du dispositif de rotation pour faire tourner le dispositif d'extraction de cellules à une vitesse angulaire prédéterminée de telle sorte qu'une force centrifuge soit exercée sur l'échantillon de fluide appliqué au dispositif d'extraction de cellules.
